(19) Europäisches Patentamt
European Patent Office
Office européen des brevets

(11) EP 1 665 983 B1

(12) **EUROPÄISCHE PATENTSCHRIFT**

(45) Veröffentlichungstag und Bekanntmachung des Hinweises auf die Patenterteilung:
**25.02.2015 Patentblatt 2015/09**

(51) Int Cl.:
***A61B 5/053*** *(2006.01)*   ***A61B 5/00*** *(2006.01)*

(21) Anmeldenummer: **05024364.1**

(22) Anmeldetag: **09.11.2005**

(54) **Vorrichtung zum Bestimmen der Thorax-Impedanz**

Device for determining impedance of the thorax

Appareil de détermination de l'impédance du thorax

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **02.12.2004 DE 102004059082**

(43) Veröffentlichungstag der Anmeldung:
**07.06.2006 Patentblatt 2006/23**

(73) Patentinhaber: **Biotronik CRM Patent AG 6340 Baar (CH)**

(72) Erfinder:
• **Czygan, Gerald, Dr.**
**91054 Buckenhof (DE)**
• **Lippert, Michael, Dr.**
**91522 Ansbach (DE)**

(74) Vertreter: **Lindner-Vogt, Karin L. Biotronik SE & Co. KG Woermannkehre 1 12359 Berlin (DE)**

(56) Entgegenhaltungen:
EP-A- 0 985 429    US-A- 5 522 860
US-A1- 2004 102 712    US-B1- 6 473 640

• PALKO, T ET. AL.: "Multifrequency device for measurement of the complex electrical bio-impedance - design and applcation" PRCEEDINGS RC IEEE - EMBS & 14TH BMESI, [Online] 1995, Seiten 145-146, XP002489921 Gefunden im Internet: URL:http://ieeexplore.ieee.org/iel3/3804/1 1132/00508682.pdf> [gefunden am 2008-07-28]

**Beschreibung**

Die Erfindung betrifft ein elektromedizinisches Implantat wie zum Beispiel einen Herzschrittmacher, Kardioverter, Defibrillator oder ein reines Monitoring-Implantat, das über Mittel zum Erfassen der Impedanz von menschlichem oder tierischem Gewebe verfügt.

Außerdem betrifft die Erfindung ein System mit wenigstens einem solchen elektromedizinischen Implantat, das zusätzlich mit einer drahtlosen Datenschnittstelle versehen ist, und einem Patientengerät, einem Home Monitoring Service Center oder beidem.

Aus dem Dokument US6473640 ist der allgemeine Teil des Anspruchs 1 bekannt, nämlich:

Ein elektromedizinisches Implantat mit wenigstens

- einem Messsignalgenerator,
- einer Impedanzmesseinheit zur Bestimmung der Impedanz von menschlichem Gewebe, insbesondere Lungengewebe,
- einer Elektrodenanordnung mit mindestens zwei Elektroden die direkt oder mittelbar mit dem Messsignalgenerator und der Impedanzmesseinheit verbunden sind oder wenigsten zeitweilig verbunden werden können, sowie
- einer Kontrolleinheit die zum Steuern von Messsignalgenerator und Impedanzmesseinheit mit dem Messsignalgenerator und der Impedanzmesseinheit wenigstens mittelbar verbunden ist und welche ausgebildet ist:

   zu wenigstens zwei unterschiedlichen Zeitpunkten den Messsignalgenerator zu veranlassen, einen Strompuls zu erzeugen und abzugeben, so dass die Abgabe des ersten Strompulses vor dem Beginn der Abgabe des zweiten Strompulses beendet ist und die Pulsdauern der abgegebenen Strompulse sich voneinander unterscheiden und welche zudem ausgebildet ist,

   die Impedanzmesseinheit zu veranlassen, die zwischen den mit dem Messsignalgenerator und der Impedanzmesseinheit verbundenen Elektroden anliegende Spannung zu Beginn einer bestimmten, mit dem Ende der Abgabe des jeweiligen Strompulses endenden Zeitspanne zu messen und als einen die gemessene Spannung repräsentierenden Spannungswert auszugeben

In der Erfindung wird im Wesentlichen der kapazitive Anteil der Lungenimpedanz bestimmt, weshalb das erwartete Ergebnis die Charakteristik einer Lade- bzw. Entladekurve aufweist. Deshalb ist die Kontrolleinheit des elektromedizinischen Implantates dazu ausgebildet, zu jedem Messpunkt gemäß der bekannten Formel für das Aufladen eines Kondensators Ausgleichskurvenwerte zu berechnen, deren Wert insbesondere von den Parametern $\tau$, der Zeitkonstante, und $U_0$, der Ladespannung, abhängen. Die Kontrolleinheit bestimmt dabei für eine Vielzahl von Parameterkombinationen mögliche Ausgleichskurvenwerte oder greift auf eine in einem nichtflüchtigen Speicher abgelegte Tabelle solcher Werte zu und vergleicht diese mit den tatsächlich gemessenen Werten. Dieser Vergleich wird durch die Bestimmung des minimalen Betrages der Summe der Quadrate der Differenzen zwischen Messwerten und Ausgleichskurvenwerten durchgeführt. Als Ergebnisparameterkombination wird diejenige Parameterkombination angesehen, die im Vergleich mit den tatsächlich bestimmten Messwerten die geringste Summe der Quadrate der einzelnen Abweichungen aufweist.

Aus dem Stand der Technik sind außerdem elektromedizinische Implantate mit Mitteln zur Impedanzbestimmung von menschlichem oder tierischem Gewebe zum Beispiel aus den Dokumenten US 5,957,861, US 2004/0102712 und der Veröffentlichung von Palko et. al. "Multifrequency Device for Measurement of the Complex Electrical Bio-Impedance - Design and Application" (Proceedings RC IEEE-EMBS & 14th BMESI - 1995 (1), S. 45), bekannt. Dort wird eine Vorrichtung dargelegt, die durch regelmäßiges Messen und langfristige Überwachung der Impedanz von Lungengewebe die Bildung von Lungenödemen frühzeitig zu erkennen hilft. Ödeme sind Flüssigkeitsansammlungen, die insbesondere bei Patienten mit chronischem Herzversagen auftreten. Der Körper reagiert auf das verminderte Pumpvermögen des Herzens mit Kompensationsmechanismen, die jedoch langfristig zu einer fortschreitenden Verschlechterung des Herzzustandes führen. Unter anderem erhöht sich die Vorlast und es kommt zu erhöhter Flüssigkeitsretention. Der erhöhte Druck im Lungenkreislauf kann im fortgeschrittenen Zustand die Bildung von Lungenödemen hervorrufen.

Die Impedanzmessung des Lungengewebes macht sich den Effekt zu Nutzen, dass mit Flüssigkeit gefülltes Körpergewebe einen anderen Leitwert aufweist als gesundes Gewebe. Die Erfahrung zeigt, dass solche Impedanzmessungen eine frühere Diagnose eines Lungenödems erlauben als traditionelle Verfahren. Elektromedizinische Implantate, die durch Impedanzmessungen einem Arzt solche Daten zur Verfügung stellen, die die Diagnose eines Lungenödems erlauben, genießen deshalb einige Aufmerksamkeit in der Entwicklung. Insbesondere als vorteilhaft hat sich dabei die Integration der Impedanzmessung in einen Herzschrittmacher erwiesen.

Herzschrittmacher werden gewöhnlich unterhalb des Schlüsselbeins auf der dem Herzen gegenüberliegenden, aus Sicht des Arztes linken Köperseite implantiert und verfügen über eine Elektrode, die im Herzgewebe des Patienten verankert ist. In dieser Konstellation liegt der Hauptteil des Lungengewebes des Patienten zwischen dem Herzschrittmachergehäuse und der Elektrode im Herzen. Wird nun durch die Herzelektrode ein geringer Strom injiziert, der zu schwach ist, um einen Herzschlag oder eine sonstige Reaktion des Körpers hervorzurufen, kann gleichzeitig die zwischen Herzschrittmachergehäuse und Elektrode anliegende Spannung gemessen werden. Diese Spannung ist, bezogen auf den Wert des initiierten Stromes, ein Indikator für den Zustand des Lungengewebes des Patienten. Dabei genügt jedoch nicht die Betrachtung des Absolutwertes der gemessenen Impedanz, da diese in hohem Maße von der Anatomie des Patienten und der Qualität des Kontaktes zwischen Herzschrittmacher-Elektrode und Körpergewebe abhängig ist. Vielmehr müssen die gemessenen Impedanzwerte über längere Zeit gespeichert und mit älteren Werten verglichen werden. Treten innerhalb von Tagen oder Wochen Veränderungen des gemessenen Impedanzwertes auf, kann dies ein Hinweis auf die Bildung eines Lungenödems sein.

Handelsübliche Herzschrittmacher und sonstige elektromedizinische Implantate verfügen gewöhnlich über eine drahtlose Datenschnittstelle, über die der Herzschrittmacher medizinische und technische Daten an ein externes Gerät übermitteln kann. Wenn eine langreichweitige Telemetrie im Schrittmacher integriert wurde, können die von einem solchen Herzschrittmacher ermittelten Impedanzwerte des Lungengewebes auf diese Weise an ein Home Monitoring Service Center übermittelt werden, wo die Daten einem betreuenden Arzt zum Stellen einer Diagnose zur Verfügung gestellt werden. Befindet der betreuende Arzt aufgrund der übermittelten Daten, dass die Gefahr einer Lungenödembildung gegeben ist, kann der Patient lange vor dem Auftreten physischer Beschwerden zur Behandlung bestellt werden.

Die in US 5,957,861 beschriebene Vorrichtung hat jedoch den Nachteil, dass langfristige Veränderungen der gemessenen Impedanzwerte auch aufgrund anderer Ursachen entstehen können, wie z.B. der Vernarbung des den Herzschrittmacher und die Herzschrittmacherelektrode umgebenden Gewebes, oder einer Änderung der Herzgeometrie aufgrund fortschreitenden Herzversagens.

Im Hinblick auf den Stand der Technik ergibt sich somit die Aufgabe, eine Vorrichtung einzuführen, die eine zuverlässigere Bestimmung der Lungenimpedanz unabhängig von sonstigen langfristigen Veränderungen erlaubt.

Diese Aufgabe wird durch die erfindungsgemäße Vorrichtung dadurch gelöst, dass die Lungenimpedanz so gemessen wird, dass insbesondere die durch das Lungengewebe verursachten Komponenten der Impedanz den Messwert bestimmen.

Der Erfindung liegt die Erkenntnis zugrunde, dass der kapazitive Anteil der zu bestimmenden Impedanz zu einem großen Teil auf die Lunge zurückzuführen ist, während die ohmschen Anteile durch die Gesamtheit des Körpergewebes bestimmt werden. Dementsprechend beruht die Erfindung auf dem Gedanken, dass die erfindungsgemäße Vorrichtung den gemessenen Impedanzwert in seine Komponenten zerlegen können soll, um Fehlalarme oder, schlimmer noch, eine Verdeckung der Symptome einer tatsächlich vorhandenen Ödembildung aufgrund der genannten oder anderer Effekte ausschließen zu können.

Wird das zwischen Herzschrittmacherelektrode und Herzschrittmachergehäuse liegende Körpergewebe in ein elektrisches Ersatzschaltbild überführt, so ergibt sich dieses in Näherung als Serienschaltung eines ohmschen Widerstandes, der die Kontaktwiderstände der Messelektroden und das Herz- und sonstige Gewebe repräsentiert, und eines sogenannten RC-Gliedes, dass das Lungengewebe darstellt.

Der Gegenstand der hier vorliegenden Erfindung vermag aufgrund ihrer neuartigen Messmethode Impedanzänderungen von Gewebe außerhalb der Lunge (vorwiegend ohmsche Komponenten) zu unterdrücken, und damit selektiv Impedanzänderungen der Lunge herauszustellen, indem sie den kapazitiven Teil der Impedanz berücksichtigt.

Zur Impedanzmessung verfügt das erfindungsgemäße elektromedizinische Implantat in jedem Falle über einen Messsignalgenerator, eine Impedanzmesseinheit, eine Kontrolleinheit sowie wenigstens zwei Elektroden oder einen Anschluss für Elektroden, von denen eine die zur Herzstimulation verwendete Herzelektrode und die andere das Gehäuse des elektromedizinischen Implantates als Referenzelektrode sein können. Die Elektroden bzw. deren Anschlüsse sind direkt oder mittelbar mit dem Messsignalgenerator und der Impedanzmesseinheit verbunden oder verbindbar. Sowohl der Messsignalgenerator als auch die Impedanzmesseinheit sind wiederum mit der Kontrolleinheit verbunden.

Es sind auch Ausführungen des elektromedizinischen Implantates vorgesehen, die über mehr als zwei Elektroden verfügen, wobei diese beliebige im Stand der Technik bekannte und für die Messung geeignete Elektroden sein können. Sind mehr als zwei Elektroden vorgesehen, kann das elektromedizinische Implantat dazu ausgebildet sein, mit im Betrieb veränderlich vorgebbaren Kombinationen von Elektroden betrieben zu werden, so dass Messsignalgenerator und Impedanzmesseinheit mit unterschiedlichen Elektroden verbunden werden können.

In der erfindungsgemäßen Lehre ist der Messsignalgenerator dazu ausgebildet, einen Strompuls oder eine Folge von Strompulsen zu erzeugen und abzugeben. Besonders bevorzugt werden biphasische Strompulse verwendet, wie weiter unten erläutert wird. Die Impedanzmesseinheit bestimmt dabei die Impedanz, indem sie die halbe Spannungsdifferenz der während zweier gegenpoliger Strompulse gemessenen Spannungen bestimmt. Dies hat den Vorteil, dass durch diese differentielle Messweise externe, über die Messdauer konstante Störspannungen (elektrochemisches Potential, IEGM) unterdrückt werden. Während der Abgabe eines solchen Strompulses bestimmt die Impedanzmesseinheit des elektromedizinischen Implantates wenigstens zu zwei unterschiedlichen Zeitpunkten die zwischen den Messelektroden anliegende Spannung und gibt einen die jeweils gemessene Spannung repräsentierenden Spannungswert, der auch eine digital kodierte Zahl sein kann, aus.

Die Differenz der beiden während der Abgabe eines Strompulses gemessenen Spannungen entsteht aufgrund der Umladung der kapazitiven Impedanzanteile des Lungengewebes und liefert einen verbesserten Indikator für den Zustand des Patienten. Die im elektrischen Ersatzschaltbild des Körpergewebes in Serie mit dem Lungengewebe liegenden ohmschen Widerstände werden durch die Differenzbildung der beiden während der Abgabe eines Strompulses gemessenen Spannungswerte auf einfache Weise eliminiert. Dadurch wird das Messergebnis auch nicht aufgrund von langfristigen Veränderungen dieser ohmschen Widerstände, etwa durch Narbenbildung an den Kontaktstellen des elektromedizinischen Implantates, verfälscht.

In einer Umsetzung des elektromedizinischen Implantates ist der Messsignalgenerator ausgebildet, Strompulse variabler Pulsdauern zu erzeugen. Die Kontrolleinheit des elektromedizinischen Implantates veranlasst die Abgabe wenigstens zweier unterschiedlich langer Strompulse während derer wenigstens jeweils eine Messung durch die Impedanzmesseinheit durchgeführt wird. Diese Messungen finden jeweils mit einem festen Abstand zum Zeitpunkt des Endes des jeweiligen Strompulses statt. Wegen der unterschiedlichen Dauer der Strompulse ist bei den beiden Messungen folglich eine unterschiedlich lange Zeit seit Beginn der Abgabe des Strompulses verstrichen, so dass die kapazitiven Elemente der Lungenimpedanz unterschiedlich lang aufgeladen wurden. Durch die unterschiedlich langen Ladezeiten ergeben sich unterschiedliche Messspannungen, deren Differenz wiederum einen besseren Indikator für den gegenwärtigen Zustand des Lungengewebes abgibt als der reine Impedanzwert selbst.

Bei beiden Umsetzungen des Erfindungsgedankens können mehr als zwei Messungen pro Messzyklus durchgeführt werden. Im ersten Fall geschieht dies durch mehr als zwei Messungen während der Abgabe eines einzelnen Strompulses, bei der zweiten Alternative durch die Abgabe von mehreren verschieden langen Strompulsen und der Durchführung einer Messung pro Strompuls.

Bei einer unabhängigen und alternativen Umsetzung der Erfindung ist der Messsignalgenerator dazu ausgebildet, Sinusströme unterschiedlicher Frequenzen zu erzeugen.

Aufgrund des unterschiedlichen Frequenzverhaltens von Herz- und Lungengewebe erzeugen zwei Sinusströme gleicher Amplitude, aber unterschiedlicher Frequenz, die in das Körpergewebe injiziert werden, an den Messelektroden unterschiedlich starke Sinusspannungen. So ist zum Beispiel bekannt, dass der spezifische Widerstand von Lungengewebe für eine zehnfach höhere Frequenz auf etwa 60% des Wertes bei der niedrigeren Frequenz abfällt, wo hingegen der spezifische Widerstand von Herzgewebe nur um ca. 5% abfällt.

Aus der Literatur sind folgende Werte bekannt: p(10 kHz)/p(100 kHz) ergibt für Lungengewebe etwa 1,7, für Herzgewebe 1,05, wobei p(f) der spezifische Widerstand des Gewebes bei der Frequenz f ist.

Dieser Umsetzung des erfinderischen Gedankens liegt die Lehre zugrunde, dass sich aus zwei Impedanzmessungen bei unterschiedlichen Frequenzen durch Subtraktion des Impedanzwertes für die Messung mit einem Messsignal höherer Frequenz vom Impedanzwert der Messung mit niedriger Frequenz ein überwiegend von der Impedanz des Herzgewebes unabhängiger Messwert ergibt, weshalb die sich aus dem Stand der Technik ergebende Aufgabe, einen verbesserten Indikator für die Bildung von Lungenödemen zur Verfügung zu stellen, auch durch die dritte Umsetzungsweise gelöst wird.

Hierzu ein Beispiel: setzt man näherungsweise voraus, dass die Impedanzen von Lungengewebe und Herzgewebe in Reihe geschaltet sind, ergibt sich die Gesamtimpedanz $Z_{ges}$ zu $Z_{ges} = Z_{Herz} + Z_{Lunge}$. Da $Z(10 \text{ kHz}) = p(10 \text{ kHz})/p(100 \text{ kHz}) * Z(100 \text{ kHz})$ ist, folgt:

$$Z(10 \text{ kHz}) - Z(100 \text{ kHz}) = 0{,}05 * Z_{Herz}(100 \text{ kHz}) + 0{,}7 * Z_{Lunge}(100 \text{ kHz})$$

Werden folglich die für zwei unterschiedliche Frequenzen gemessenen Impedanzen subtrahiert, ist das Ergebnis in hohem Maße von der Impedanz der Lunge abhängig, jedoch nur in geringem Maße von der die Qualität der Messung beeinträchtigenden Herzimpedanz (Verhältnis 14:1).

Ein weiterer Aspekt der Erfindung richtet sich auf ein System, das neben einem elektromedizinischen Implantat der dargelegten Art über ein Patientengerät oder ein Home Monitoring Service Center oder beides verfügt.

Moderne elektromedizinische Implantate, insbesondere Herzschrittmacher, Defibrillatoren und dergleichen, bieten Arzt und Patienten ein Höchstmaß an Sicherheit und Komfort durch sogenannte Home Monitoring Funktionen.

Dabei protokolliert das Implantat Diagnose- und Therapieinformationen und überträgt diese Informationen über eine drahtlose Datenschnittstelle an ein externes Patientengerät, das der Patient nach ärztlicher Vorgabe mit sich führt. Von dort werden die Daten z.B. über ein Mobiltelefonnetz an das Home Monitoring Service Center weitergeleitet, wo sie für den Arzt gespeichert und visualisiert werden. Der Arzt kann sich auf diese Weise direkt über den Therapieverlauf und den aktuellen Gesundheitszustand seiner Patienten informieren und bekommt die Möglichkeit, schnell auf eventuelle Gesundheitsveränderungen zu reagieren. Die mittelbare Übertragung der Patientendaten über das Patientengerät bringt neben weiteren Vorteilen vor allem den des geringeren Leistungsverbrauchs für die Übertragung seitens des Implantates mit sich, dessen Batterien nur operativ gewechselt werden können.

Ohne Home Monitoring kann der Arzt diese Informationen nur im Rahmen einer Untersuchung des Patienten abfragen. In kritischen Situationen würde dies zu unerwünschten Verzögerungen im Informationsfluss führen. Zudem ist jede Untersuchung für Arzt und Patient mit erheblichem Zeitaufwand verbunden. Häufige Untersuchung beeinträchtigen Mobilität und Lebensqualität des Patienten.

Beim Home Monitoring werden die Implantatinformationen über das Patientengerät (siehe auch US 6553262, US 5752976) im Hintergrund verschickt, ohne dass der Patient in der normalen Lebensführung eingeschränkt wird; d.h., er genießt die Sicherheit des ärztlichen Monitorings ohne die Belastung von häufigen Untersuchungen.

Das beanspruchte System aus neuartigem elektromedizinischem Implantat und Patientengerät und/oder Home Monitoring Service Center erweitert die bekannten Vorteile solcher Systeme durch die Verfügbarkeit verbesserter Messdaten, die die Beurteilung des Gesundheitszustandes eines Patienten durch seinen betreuenden Arzt erleichtern.

Das elektromedizinische Implantat verfügt über eine Kontrolleinheit, die dazu ausgebildet ist, die Differenz der gemessenen Spannungswerte zweier zu unterschiedlichen Zeitpunkten durchgeführter Spannungsmessungen zu bestimmen. Diese beiden Spannungsmessungen werden dabei zu unterschiedlichen Zeitpunkten während der Abgabe eines Strompulses oder während zweier verschiedener Strompulse mit unterschiedlichem zeitlichen Abstand zum Beginn der Ausgabe der Strompulse durchgeführt. Besonders bevorzugt werden die beiden Spannungsmessungen während der Abgabe zweier gegenpoliger Strompulse durchgeführt, wobei bei der Differenzbildung die Vorzeichen der gemessenen Spannungen berücksichtigt werden. Durch diese biphasische Spannungsmessung werden externe Spannungen (elektrochemisches Potential, Ladung auf der Elektrode, IEGM) unterdrückt. Durch die Differenzbildung der bei verschiedenen Pulsdauern durchgeführten biphasischen Spannungsmessungen wird der zeitlich invariante Teil der beiden Messspannungen aus dem Ergebnis eliminiert, wodurch dieses einen besonders guten Indikator für den Zustand des Lungengewebes abgibt.

Das Implantat ist dazu ausgebildet, mehrere Messungen während der Abgabe eines einzigen Strompulses oder während der Abgabe mehrere Strompulse mit verschiedenem zeitlichen Abstand zum Beginn der Abgabe des jeweiligen Pulses durchzuführen. In jeder realen Messanordnung werden Messungen immer durch Störeinflüsse wie Rauschen oder elektromagnetische Störfelder verfälscht. Werden mehr als zwei Messungen durchgeführt, kann der Einfluss dieser Fehlerquellen durch mathematische Ausgleichsverfahren verringert werden. Dabei steigt die Qualität des Messergebnisses mit der Anzahl der durchgeführten Messungen.

In der vorliegenden Erfindung wird im Wesentlichen der kapazitive Anteil der Lungenimpedanz bestimmt, weshalb das erwartete Ergebnis die Charakteristik einer Lade- bzw. Entladekurve aufweist. In dieser Ausführung ist die Kontrolleinheit des elektromedizinischen Implantates deshalb dazu ausgebildet, zu jedem Messpunkt gemäß der bekannten Formel für das Aufladen eines Kondensators Ausgleichskurvenwerte zu berechnen, deren Wert insbesondere von den Parametern $\tau$, der Zeitkonstante, und $U_0$, der Ladespannung, abhängen. Die Kontrolleinheit bestimmt dabei für eine Vielzahl von Parameterkombinationen mögliche Ausgleichskurvenwerte oder greift auf eine in einem nichtflüchtigen Speicher abgelegte Tabelle solcher Werte zu und vergleicht diese mit den tatsächlich gemessenen Werten. Dieser Vergleich wird besonders bevorzugt durch die Bestimmung des minimalen Betrages der Summe der Quadrate der Differenzen zwischen Messwerten und Ausgleichskurvenwerten durchgeführt. Als Ergebnisparameterkombination wird diejenige Parameterkombination angesehen, die im Vergleich mit den tatsächlich bestimmten Messwerten die geringste Summe der Quadrate der einzelnen Abweichungen aufweist.

Ist das elektromedizinische Implantat ausgebildet, mehrere unterschiedlich lange Strompulse auszugeben und pro Strompuls nur eine Spannungsmessung durchzuführen, werden die für jeden Strompuls bestimmten Messwerte nach dem zeitlichen Abstand der Messung zum Beginn der Ausgabe des jeweiligen Strompulses sortiert und anschließend dem gleichen Ausgleichsverfahren zugeführt.

Da die Berechnung von Exponentialfunktionen sehr rechenaufwendig ist, kann eine Ausgleichskurve der Messwerte alternativ auch durch eine Approximation durch ein Polynom berechnet werden. In diesem Fall kann das bekannte Verfahren der kleinsten Fehlerquadrate verwendet werden, das sich für Polynome mit geringem Rechenaufwand durchführen lässt.

Der Fachmann wird in jedem Fall erkennen, dass ihm eine große Bandweite von mathematischen Fehlerreduktionsverfahren zur Verfügung steht, die die Berechnung eines verbesserten Gesamtmessergebnisses auf der Grundlage der ursprünglichen Messergebnisse ermöglichen.

Da die bekannte exponentielle Ladefunktion zu Beginn steil und zu ihrem Ende immer flacher ansteigt, geben Messungen in ihrem Anfangsbereich besseren Aufschluss hinsichtlich der tatsächlich gegebenen Parameter, weshalb in einer vorteilhaften Umsetzung der Erfindung diese dazu ausgebildet ist, mehr Spannungsmessungen zu Beginn der Ausgabe eines Strompulses als gegen deren Ende durchzuführen. Dadurch kann der Messfehler rechnerisch minimiert werden, wodurch die Ergebnis-Parameter besonders gut geeignet sind, Veränderungen der Beschaffenheit des Lungengewebes des Patienten aufzuzeigen.

Wird die Vielzahl der Messwerte wiederum während der Ausgabe mehrerer Strompulse bestimmt, kann der Messfehler der Ergebnis-Parameter-Kombination in äquivalenter Weise reduziert werden, indem mehr Strompulse kurzer Pulsdauer als längerer Pulsdauer ausgegeben werden.

In einer Fortführung des erfinderischen Gedankens ist das elektromedizinische Implantat dazu ausgebildet anstelle der oder zusätzlich zur Ergebnisparameterkombination die Differenz zweier Ausgleichskurvenwerte, die nach dem bereits beschriebenen Verfahren bestimmt wurden, zu berechnen und als Ergebnis auszugeben. Dabei wird die Differenz der Ausgleichskurvenwerte des jeweils ersten und letzten während der Abgabe eines Strompulses gemessenen Spannungswertes bestimmt.

In einer entsprechenden Umsetzung der alternativen Messmethode bei Ausgabe mehrerer unterschiedlich langer Strompulse ist das elektromedizinische Implantat dazu ausgebildet, die Differenz der Ausgleichskurvenwerte für die Spannungsmessungen des jeweils längsten und des kürzesten Strompulses zu bestimmen. Die Bestimmung dieses Spannungsdifferenzwertes aus den Ausgleichkurvenwerten hat den Vorteil, dass ein fehlerreduzierter Messwert für die kapazitive Komponente der Gewebeimpedanz zur Verfügung gestellt wird.

Ist der Messsignalgenerator des elektromedizinischen Implantates dazu ausgebildet, Strompulse zu erzeugen, so verfügt eine besonders bevorzugte Variante des elektromedizinischen Implantates über einen Messsignalgenerator, der ausgebildet ist, biphasische Strompulse zu erzeugen.

Biphasische Strompulse weisen Signalanteile positiver und negativer Auslenkung auf. Sie können an den Nulldurchgängen in zwei oder mehr Abschnitte aufgeteilt werden, die jeweils einem der beiden Vorzeichen zugehören. Bei der Anwendung in elektromedizinischen Implantaten werden insbesondere solche biphasischen Strompulse bevorzugt, bei denen sich die Integrale der positiven und der negativen Signalanteile gleichen, so dass die Differenz der beiden Integrale 0 ergibt. Biphasische Strompulse werden deshalb bevorzugt, weil sie nicht zu Elektrolyseeffekten führen oder die Verteilung von Ionen in der Körperflüssigkeit beeinflussen und deshalb medizinisch verträglicher sind. Weiterhin wird somit das "Aufladen" des Elektroden-Gewebe-Interface vermieden und die Sensing-Eigenschaften des Implantats nicht beeinflusst. Der Einfluss externer Spannungen, insbesondere solcher Gleichspannungsanteile, die nicht auf impedanzbedingtem Spannungsabfall beruhen, wie z.B. elektrochemischer Potentiale und der IEGM-Signale des Myokards, auf das Messsignal kann durch die Messung mit biphasischen Pulsen eliminiert werden.

In einer Umsetzung des Erfindungsgedankens ist der Messsignalgenerator des elektromedizinischen Implantates dazu ausgebildet, Sinusströme unterschiedlicher Frequenzen zu erzeugen und abzugeben. Der Impedanzmesseinheit fällt dann die Aufgabe zu, die Amplitude der bei der Abgabe eines sinusförmigen Stromes an den Messelektroden anliegenden Sinusspannung oder die Phasendifferenz zwischen sinusförmigem Messsignal und der an den Messelektroden anliegenden Sinusspannung zu bestimmen. Sowohl das Verhältnis der Amplituden als auch das Phasenverhältnis von Messsignal und gemessener Sinusspannung können Aufschluss über den kapazitiven Anteil der Gewebeimpedanz geben.

Die Amplitudenmessung wird bevorzugt über Mittelung der Messspannung in einem geeigneten Filter oder einer Phase-Locked Loop (PLL) und anschließender Messung der resultierenden Gleichspannung durchgeführt, die Messung des Phasenverhältnisses durch Detektion der Nulldurchgänge von Messsignal und gemessenem Signal und Bestimmen der Zeiten, die zwischen den Nulldurchgängen liegen.

In einer Ausführung der Erfindung kann diese dazu ausgebildet sein, die Frequenz des erzeugten sinusförmigen Messsignals mit der Zeit zu erhöhen, bis die Impedanzmesseinheit eine bestimmte, vorgebbare Phasendifferenz feststellt. Zur Verbesserung der Messsicherheit kann in einer Abwandlung dabei ein Zeitraum vorgegeben werden, über den die vorgebbare Phasendifferenz festgestellt werden soll. Alternativ kann die Impedanzmesseinheit auch dazu ausgebildet sein, maximale oder minimale Phasendifferenzen zwischen Messsignal und gemessenem Signal während eines vorgebbaren Zeitraumes zu detektieren.

Die Qualität der Messung kann verbessert werden, indem die Messspannung zuvor geeignet gefiltert wird, wodurch Störsignale anderer Frequenzen eliminiert oder unterdrückt werden können. Eine bevorzugte Ausführung dieser alternativen Form des elektromedizinischen Implantates verfügt deshalb über ein Filter, das zwischen Messelektrode und Impedanzmesseinheit geschaltet ist und als Tiefpass- oder Bandpassfilter ausgeführt ist. Der Durchlassbereich des Filters ist dabei so gewählt, dass die Frequenz der vom Messsignalgenerator erzeugten Sinusströme in ihn fällt.

In einer besonders vorteilhaften Fortführung dieser bevorzugten Ausführung des elektromedizinischen Implantates ist der Durchlassbereich des Filters kleiner als die Differenz der Frequenzen des höchst- und des niedrigstfrequenten Sinusstromes, der vom Messsignalgenerator abgegeben wird. Damit jedoch das Filter die jeweiligen Messsignale passieren lässt, ist der Durchlassbereich zusätzlich noch veränderbar gestaltet, so dass bei jeder Einzelmessung etwaige Störeinflüsse besonders breitbandig unterdrückt werden.

In einer weiteren Ausführung verfügt das elektromedizinische Implantat über eine Subtraktionseinheit, die dazu ausgebildet ist, eine Differenz der durch die Impedanzmesseinheit für unterschiedliche Sinusströme bestimmten Amplitudenspannungen zu bestimmen und als Amplitudendifferenzwert auszugeben. Auf diese Weise wird von der Subtraktionseinheit ein Ergebniswert zur Verfügung gestellt, der überwiegend von der Impedanz des Lungengewebes abhängig ist und somit einen besonders guten Indikator für den Zustand des Lungengewebes darstellt.

Gemäß der dieser alternativen Umsetzung der Erfindung zugrundeliegenden Lehre werden für die Bestimmung der Lungenimpedanz Sinusströme einer Frequenz zwischen ungefähr 1 kHz und ungefähr 100 kHz besonders bevorzugt.

Frequenzen zwischen 2 und 20 kHz sind technisch leicht beherrschbar und die Eigenschaften von Körpergewebe bei einer Injektion von Strömen dieser Frequenzen hinreichend bekannt.

Zwischen Messelektrode und Impedanzmesseinheit kann vorteilhafterweise ein Verstärker geschalten werden, der dazu ausgebildet ist, die zwischen den Messelektroden anliegende Messspannung zu verstärken oder abzuschwächen und die resultierende Spannung der Impedanzmesseinheit zuzuleiten. Indem der Verstärkungsfaktor des Verstärkers veränderbar gestaltet wird, kann das elektromedizinische Implantat die an der Impedanzmesseinheit anliegende Spannung dergestalt an den Messbereich der Impedanzmesseinheit anpassen, dass eine möglichst genaue Messung durchgeführt werden kann. In dieser Variante des elektromedizinischen Implantates wird der Messwert der Impedanzmessung auf den Verstärkungsfaktor des Verstärkers bezogen. Ist der Verstärkungsfaktor des Verstärkers im Betrieb dergestalt anpassbar, dass der Betrag des Maximalwertes der verstärkten Spannung einen ersten Spannungswert nicht unter- und einen zweiten Spannungswert nicht überschreitet, ist außerdem gewährleistet, dass die Impedanzmesseinheit nicht übersteuert wird, was grobe Messfehler zur Folge hätte.

Da die von der Impedanzmesseinheit zu bestimmende Spannung bzw. Amplitude gewöhnlich einen zeit- bzw. frequenzinvarianten Anteil aufweist, verfügt eine weitere vorteilhafte Variante über eine Subtraktionseinheit, die dem Verstärker vorgeschaltet ist. Diese Subtraktionseinheit ist dazu ausgebildet, vor der Messung der Messspannung durch die Impedanzmesseinheit diese um einen konstanten, einstellbaren Wert zu verringern, so dass der Impedanzmesseinheit möglichst nur der zeitvariante bzw. frequenzvariante Teil der Messspannung zugeführt wird. Auf diese Weise wird der Messfehler bei der Bestimmung des kapazitiven Impedanzanteils minimiert.

Wird die Messung der Gewebeimpedanz nur zwischen zwei Punkten im Körper vorgenommen, erfasst der resultierende Messwert unterschiedliche Geweberegionen in unterschiedlicher Gewichtung, da der Messstrom bevorzugte Wege durch das Gewebe wählt. Werden mehr als zwei Elektroden vorgesehen, kann die Qualität der Impedanzmessungen dadurch verbessert werden, dass die Impedanz zwischen unterschiedlichen Kombinationen von Elektroden bestimmt wird. Der Messstrom fließt dabei aufgrund der unterschiedlichen Positionierung der Elektroden im Körper auf unterschiedlichen Wegen, weshalb mehrere für unterschiedliche Elektrodenanordnungen durchgeführte Messungen die Gesamtheit des Lungengewebes besser zu berücksichtigen vermögen. Deswegen sind bei Ausführungen des elektromedizinischen Implantates mit mehr als zwei Elektroden solche Ausführungen bevorzugt, bei denen Impedanzmesseinheit und Messsignalgenerator mit unterschiedlichen Elektroden verbunden werden können.

Vorteilhafter Weise ist die Kontrolleinheit von elektromedizinischen Implantaten mit mehr als zwei Elektroden dazu ausgebildet, die für unterschiedliche Elektrodenanordnungen bestimmten Messwerte in einen die verschiedenen Messwerte repräsentierenden Mittelwert zu überführen. Wird dieser Mittelwert als Ergebnis ausgegeben, ermöglicht er eine kompaktere Darstellung und Übermittlung als verbesserter Indikator für den Zustand des Lungengewebes des Patienten.

Alle beanspruchten Ausführungen eines elektromedizinischen Implantates mit Mitteln zur Impedanzbestimmung von Körpergewebe sind besonders bevorzugt als Herzschrittmacher, Kardioverter oder Defibrillator ausgeführt. Die typische Positionierung solcher elektromedizinischer Implantate und ihrer Elektroden erlaubt eine besonders einfache Integration der Mittel zur Impedanzbestimmung von Körpergewebe, die insbesondere bei Patienten mit Herzinsuffizienz besonders sinnvoll sind, weil diese in hohem Maße der Gefahr der Ödembildung ausgesetzt sind.

Da die Messung der Gewebeimpedanz aufgrund der Positionierung der Herzelektrode auch vom Kontraktionszustand des Herzens und vom Blutgehalt der Blutgefäße und Kapillaren abhängig ist, wird in einer Ausführung des elektromedizinischen Implantates vorteilhafterweise ein Herzaktivitätsdetektor vorgesehen, der ausgebildet ist, den Herzzyklus eines Patienten zu messen. Indem der Herzaktivitätsdetektor den Herzzyklus eines Patienten bestimmt, können die Strompulse vom Messsignalgenerator zu festen Zeitpunkten innerhalb eines Herzzyklus abgegeben werden, so dass für Messungen zu unterschiedlichen Zeitpunkten identische oder nahezu identische Messbedingungen sichergestellt werden können.

Alternativ werden während eines oder mehrerer Herzzyklen mehrere Messungen durchgeführt und die Messergebnisse anschließend gemittelt. Auf diese Weise kann ein vom jeweiligen Herzzustand weitgehend unabhängiger Impedanzwert gewonnen werden. Diese Mittelung kann auch im Patientengerät oder im Home Monitoring Service Center vorgenommen werden.

Die Impedanz des Lungengewebe hängt in hohem Maße von dem in der Lunge enthaltenen Luftvolumen ab, weshalb eine besonders bevorzugte Ausführung des elektromedizinischen Implantates über einen Atemaktivitätsdetektor verfügt, der ausgebildet ist, den Atemzyklus eines Patienten zu messen. Durch den Atemaktivitätsdetektor kann das elektromedizinische Implantat Impedanzmessung zu bestimmten Zeitpunkten innerhalb eines Atemszyklus' durchführen, so dass die Zeitpunkte mehrerer Messungen jeweils beispielsweise auf den Moment des maximalen Ausatmens, des maximalen Einatmens oder eines anderen Zeitpunktes im Atemzyklus gelegt werden können. Hierdurch werden die gemessenen Impedanzwerte von dem Atemzustand weitgehend unabhängig, so dass die Vergleichbarkeit von Messwerten, die zu unterschiedlichen Zeitpunkten gewonnen wurden, verbessert wird.

Genau wie bei der Mittelung der Messergebnisse verschiedener während eines oder mehrerer Herzzyklen durchgeführter Impedanzmessungen ein vom Herzzustand unabhängiger Vergleichswert gewonnen werden kann, wird ein noch weiter verbesserter Vergleichswert gestellt, wenn sich die Mittelung auf Messergebnisse erstreckt, die während eines ganzen oder mehrerer Atemzyklen bestimmt wurden. Eine besonders bevorzugte Variante der Erfindung ist deshalb dazu ausgebildet, einen Mittelwert der Messergebnisse einer Mehrzahl während einiger Atemzyklen durchgeführten Impedanzmessungen zu berechnen. Diese Mittelung kann auch im Patientengerät oder im Home Monitoring Service Center vorgenommen werden.

Anstelle der Bestimmung des Mittelwertes kann auch der Median der Messergebnisse einer Reihe von Impedanzmessungen treten. Der Median hat den Vorteil, dass er mit geringerem Rechenaufwand bestimmt werden kann und dass sein Wert nicht durch extreme Messwerte, die auf Messfehlern beruhen könnten, verzerrt wird.

Alle Varianten des erfindungsgemäßen elektromedizinischen Implantates verfügen vorzugsweise über eine drahtlose Datenübertragungsschnittstelle, die dazu ausgebildet ist, neben anderen medizinischen oder technischen Daten die durch die Kontrolleinheit bestimmten Ergebniswerte wie Spannungsdifferenzwert, Mittelwert, Wert des ersten Parameters $U_0$, Wert des zweiten Parameters $\tau$ und Amplitudendifferenzwert und/oder einen oder mehrere der von der Impedanzmesseinheit bestimmten Spannungs- bzw. Amplitudenwerte über die drahtlose Datenübertragungsschnittstelle direkt oder mittelbar über ein Patientengerät an ein Home Monitoring Service Center zu senden. Alle diese Daten sind dabei vorzugsweise als binäre Digitalzahlen kodiert. Durch die drahtlose Datenübertragung der Patientendaten kann dem behandelnden Arzt der Zugriff auf aktuelle, den Zustand des Lungengewebes des Patienten indizierende Messwerte eingeräumt werden, ohne dass der Patient sich dazu in ein Krankenhaus begeben müsste.

In einer Variante des beanspruchten Systems mit einem erfindungsgemäßen elektromedizinischen Implantat und einem Patientengerät und einem Home Monitoring Service Center sind das Patientengerät oder das Home Monitoring Service Center dazu ausgebildet, Kurzzeit- und Langzeitmittelwerte der einzelnen vom elektromedizinischen Implantat empfangenen Patientendaten zu berechnen. Diese Mittelwerte liefern einen guten Vergleichswert zur Beurteilung der zuletzt empfangenen Patientendaten.

In einer weiteren Variante sind das Patientengerät oder das Home Monitoring Service Center dazu ausgebildet, empfangene Patientendaten mit den berechneten Kurzzeit- und Langzeitmittelwerten, etwa durch Subtraktion der Mittelwerte von den Patientendaten, zu vergleichen.

Eine bevorzugte Variante des Systems ermöglicht es dem behandelnden Arzt, eine Vielzahl von Patienten bequem zu betreuen, indem es ausgebildet ist, Abweichungen der mit den Mittelwerten verglichenen Patientendaten anzuzeigen. Dabei können besonders starke Abweichungen gesondert hervorgehoben werden.

Im Folgenden soll die Erfindung anhand von Abbildungen von Ausführungsbeispielen und weiteren erläuternden Abbildungen beschrieben werden.

Abbildung 1 zeigt modellhaft ein elektrisches Ersatzschaltbild des Körpergewebes zwischen den beiden Messelektroden im Betriebsfall.

Abbildung 2 zeigt in seinen vier Unterabbildungen a, b, c und d mehrere unterschiedliche Spannungsverläufe, wie sie sich bei der Injektion eines biphasischen Strompulses in das Körpergewebe ergeben können.

Abbildung 3 zeigt ein Blockdiagramm eines Ausführungsbeispiels der Erfindung.

Abbildung 4 zeigt drei Koordinatensysteme, die mögliche Strom- und Spannungsverläufe während der Durchführung des Messverfahrens zeigen.

Abbildung 5 zeigt ein weiteres Beispiel einer Folge von biphasischen Strompulsen, wie sie besonders vorteilhaft für eine differentielle Messung der Gewebeimpedanz eingesetzt werden kann.

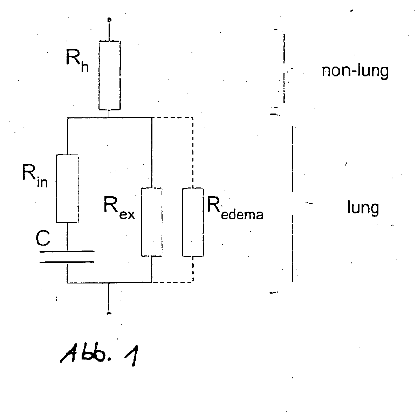

Abbildung 1 zeigt ein Netzwerk mehrerer diskreter Elemente. Es ergibt sich im Wesentlichen als Serienschaltung eines ohmschen Widerstandes $R_h$ und einer Parallelschaltung von mehreren Widerständen und einer Kapazität C. $R_h$ repräsentiert hierbei den Widerstand des Herzgewebes, in dem eine Messelektrode angeordnet ist. Der Einfachheit halber wurden sonstige Kontaktwiderstände der beiden Messelektroden mit in den Widerstand $R_h$ einbezogen. Die Parallelschaltung der Kapazität C und der ohmschen Widerstände $R_{in}$, $R_{ex}$ und $R_{edema}$ beschreibt in Näherung das Widerstandsverhalten des zwischen Herzen und elektromedizinischem Implantat liegenden Lungengewebes. $R_{in}$, $R_{ex}$ und C charakterisieren das elektrische Verhalten des gesunden Lungengewebes, während $R_{edema}$ nur hinzutritt, wenn der ohmsche Widerstand des Lungengewebes aufgrund einer Ödembildung herabgesetzt wird. Der durch das Ödem verringerte ohmsche Widerstand ist in dem elektrischen Ersatzschaltbild durch Parallelschaltung des Widerstands $R_{edema}$ berücksichtigt.

Während der zeitinvariante Teil der durch die Injektion eines Strompulses erzeugten Messspannung von der Serienschaltung des Widerstandes $R_h$ und den Widerständen $R_{in}$, $R_{ex}$ und $R_{edema}$ abhängt, wird der zeitvariante Teil der Messspannung allein von den Widerständen $R_{in}$, $R_{ex}$, $R_{edema}$ und der Kapazität C bestimmt. Die Abhängigkeit des zeitvarianten Spannungsanteils von $R_{edema}$ ist dabei wesentlich stärker als die des zeitinvarianten. Dies verdeutlicht, wie durch die Bestimmung des zeitvarianten Messspannungsanteils die Aussagekraft der gewonnenen Impedanzwerte steigt und zusätzlich Veränderungen des Widerstandes $R_h$ durch Vernarbung des die Messelektroden umgebenden Gewebes als Fehlerquelle bei der langfristigen Beobachtung der Entwicklung der Gewebeimpedanzwerte eliminiert werden kann.

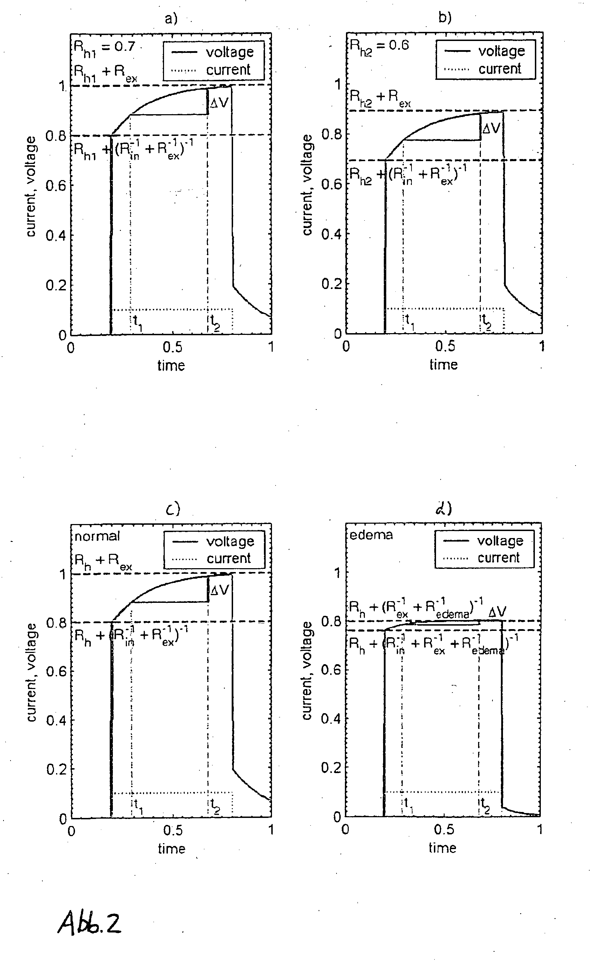

Abbildung 2 unterteilt sich in zwei Unterabbildungen, welche jede für sich wiederum in zwei weitere Teilabbildungen zerfallen. Dadurch ergeben sich insgesamt vier Teilabbildungen, die mit den Buchstaben a bis d benannt sind. Die erste Unterabbildung zeigt in den Teilabbildungen a und b zwei Spannungsverläufe, wie sie sich bei der Injektion eines rechteckigen Strompulses in das Körpergewebe ergeben könnten. Die beiden Spannungsverläufe wurden für bis auf den Wert des Widerstandes $R_h$ identische Ersatzschaltbilder des Körpergewebes berechnet. In der Unterabbildung b wurde der Wert des Widerstandes $R_h$ reduziert, was eine Abnahme des Maximalwertes des Spannungsverlaufs zur Folge hat. Deutlich erkennbar ist bei Vergleich der beiden Teilabbildungen a und b, dass sich durch die Verringerung des Widerstandes lediglich eine Abnahme des konstanten Sockelteils der Spannungskurve ergibt, während der zeitvariante obere Teil weiterhin einen identischen Verlauf aufweist. Dieses Verhalten bestätigt, dass die Differenz zweier zu unterschiedlichen Zeitpunkten $t_1$ und $t_2$ gewonnener Spannungsmesswerte einen von dem Widerstand des Herzgewebes $R_h$ unabhängigen Indikator liefern.

In der zweiten Unterabbildung der Abbildung 2 wurde bei konstantem Widerstand $R_h$ nun ein Widerstand $R_{edema}$ in das Ersatzschaltbild aufgenommen. Dieser dem Widerstand $R_{ex}$ parallel geschaltete Widerstand verringert den parallel zur Kapazität C liegenden ohmschen Widerstand, so dass diese Kapazität schneller aufgeladen beziehungsweise entladen werden kann. Während sich durch den Vergleich der beiden Spannungskurven in den Teilabbildungen c und d deutlich eine Abnahme der Amplitude des zeitvarianten Anteils der Spannungskurve zeigt, bleibt der konstante Teil der Spannungssignale trotz des Hinzutretens des Widerstandes $R_{edema}$ nahezu konstant. Es kann also gefolgert werden, dass die Differenz der beiden Messwerte zu Beginn und zum Ende der Ausgabe des Strompulses einen im Wesentlichen vom Widerstand des Herzgewebes unabhängigen, aber von der Bildung von Ödemen in hohem Maße abhängigen Indikator stellt.

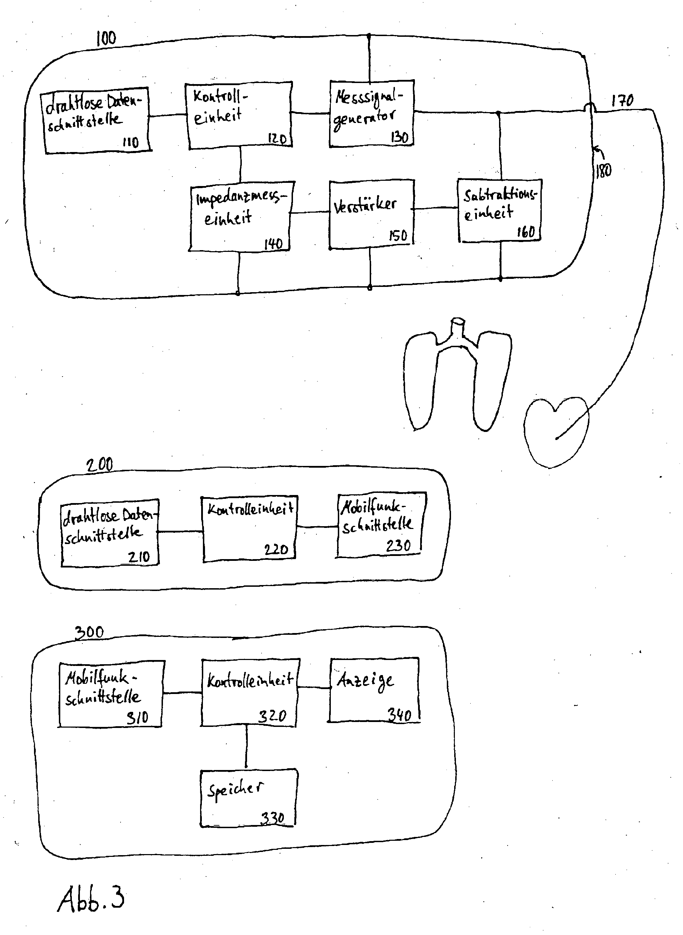

Das in Abbildung 3 gezeigt System mit einem Herzschrittmacher 100, einem Patientengerät 200 und einem Home Monitoring Service Center 300 soll als Beispiel für ein System der beanspruchten Art dienen.

Der Herzschrittmacher 100 verfügt zur Kommunikation mit dem Patientengerät 200 über eine drahtlose Datenschnittstelle 110, welche mit der zentralen Kontrolleinheit 120 des Herzschrittmachers 100 verbunden ist. Die Kontrolleinheit 120 ist mit dem Messsignalgenerator 130 und der Impedanzmesseinheit 140 verbunden und steuert diese zur Bestimmung der Impedanz des Lungengewebes. Dem Messsignalgenerator 130 fällt hierbei die Aufgabe zu, Messsignale zu erzeugen und über die angeschlossene Herzelektrode 170 auszugeben. Als Referenzelektrode dient das Gehäuse des Herzschrittmachers 100. Zwischen das Gehäuse und die Herzschrittmacherelektrode 170 ist die Subtraktionseinheit 160 geschaltet, die ausgebildet ist, von einer an den beiden Elektroden anliegenden Messspannung eine konstante Offset-Spannung abzuziehen und die resultierende Spannung dem Verstärker 150 zuzuführen. Der Verstärker 150 verstärkt die von der Subtraktionseinheit 160 empfangene Messspannung, so dass der Messbereich der Impedanzmesseinheit 140 möglichst vollständig genutzt wird, ohne die Impedanzmesseinheit 140 zu übersteuern. Die Impedanzmesseinheit 140 bestimmt zu von der Kontrolleinheit 120 festgelegten Zeitpunkten die an ihren Messeingang anliegende Messspannung und gibt einen entsprechenden Spannungsmesswert an die Kontrolleinheit 120. Die Kontrolleinheit 120 ist dabei ausgebildet, die von der Impedanzmesseinheit 140 empfangenen Spannungsmesswerte zu speichern und wenigstens die Differenz zweier aufeinanderfolgender Impedanzmessungen zu berechnen. Werden mehr als zwei Messungen pro ausgegebenem Strompuls durchgeführt oder werden mehrere verschieden lange Strompulse ausgegeben, während derer jeweils eine Impedanzmessung durchgeführt wird, ist die Kontrolleinheit 120 dazu ausgebildet, Ausgleichskurvenwerte zu berechnen, so dass ein fehlerreduzierter Ergebniswert bereitgestellt werden kann. Dieser Ergebniswert und ggf. weitere Messdaten werden von der Kontrolleinheit 120 über die drahtlose Datenschnittstelle 110 an das Patientengerät 200 gesandt.

Das Patientengerät 200 verfügt über eine drahtlose Datenschnittstelle 210, eine Kontrolleinheit 220 und eine Mobilfunkschnittstelle 230. Anstelle der Mobilfunkschnittstelle könnte auch eine Schnittstelle für eine drahtgebundene Kommunikationsform, etwa über eine gewöhnliche Telefonleitung, vorgesehen sein. Die drahtlose Datenschnittstelle 210 empfängt die von der drahtlosen Datenschnittstelle 110 des Herzschrittmachers 100 gesandten Patientendaten und gibt diese an die Kontrolleinheit 220 des Patientengeräts 200 weiter. Die Kontrolleinheit 220 leitet die empfangenen Patientendaten entweder unverändert über die Mobilfunkschnittstelle 230 an das Home Monitoring Service Center 300 weiter oder entlastet diese, indem sie bereits ansonsten der Kontrolleinheit 320 des Home Monitoring Service Centers zufallende Aufgaben vor der Übertragung der Patientendaten an das Home Monitoring Service Center 300 auf die Patientendaten anwendet.

Das Home Monitoring Service Center 300 empfängt die von dem Patientengerät 200 über ein Mobilfunknetz versandten Patientendaten über die Mobilfunkschnittstelle 310, die mit der Kontrolleinheit 320 des Home Monitoring Service Centers 300 verbunden ist. Verfügt das Patientengerät anstelle der Mobilfunkschnittstelle 230 über eine Schnittstelle für die drahtgebundene Kommunikation, ist für das Home Monitoring Service Center anstelle oder zusätzlich zu der Mobilfunkschnittstelle 310 eine drahtgebundene Datenübertragungsschnittstelle vorgesehen. Das Home Monitoring Service Center 300 verfügt in dem Beispiel zusätzlich über einen Speicher 330 sowie eine Anzeige 340. Die Kontrolleinheit 320 ist dazu ausgebildet, die empfangenen Patientendaten in dem Speicher 330 abzulegen sowie Kurzzeit- und Langzeitmittelwerte der aktuell und zuvor empfangenen Patientendaten zu berechnen. Außerdem vergleicht die Kontrolleinheit 320 die empfangenen Patientendaten mit den zuvor berechneten Kurzzeit- und Langzeitmittelwerten der Patientendaten.

Stellt die Kontrolleinheit 320 dabei eine Diskrepanz der aktuellen Messwerte von den Mittelwerten, gegebenenfalls über ein wählbares Maß hinaus fest, so bringt die Kontrolleinheit 320 diese Diskrepanz auf der Anzeigt 340 mit geeigneten optischen oder akustischen Mitteln zur Anzeige.

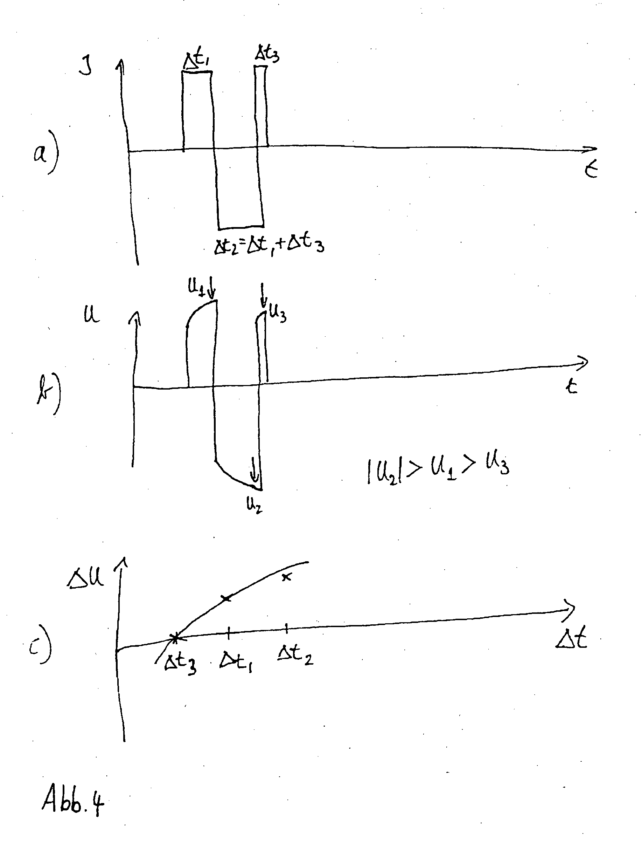

Die Abbildung 4 zeigt drei Koordinatensysteme, die mögliche Kurvenverläufe für eine Impedanzmessung darstellen. Im ersten der Koordinatensysteme ist ein biphasischer Strompuls gezeigt, der sich in drei Abschnitte, zwei positive und einen zwischen diesen beiden liegenden negativen, unterteilen lässt. Die Pulsdauer der drei Abschnitte ist dabei so gewählt, dass sich die Zeiten $\Delta t_1$ und $\Delta t_3$ genau zur Pulsdauer $\Delta t_2$ des negativen Pulses addieren. Auf diese Weise ist sichergestellt, dass die Integrale der positiven und negativen Signalanteile sich gegenseitig wegheben, was eine bessere medizinische Verträglichkeit garantiert.

Im zweiten Koordinatensystem ist ein qualitativer Verlauf einer Messspannung dargestellt, wie sie sich bei der Injektion des im ersten Koordinatensystem gezeigten biphasischen Strompulses ergeben könnte. Während der Abgabe des Strompulses werden insgesamt drei Spannungsmessungen durchgeführt, während jedes Abschnittes des biphasischen Strompulses eine. Alle drei Spannungsmessungen werden mit einem konstanten zeitlichen Abstand zum Ende des jeweiligen Abschnittes des Strompulses durchgeführt, wodurch sich für die drei Abschnitte unterschiedlich lange Abstände zum Beginn des jeweiligen Abschnittes ergeben. Aufgrund der unterschiedlichen Aufladedauern der elektrischen Kapazität C der Lunge liefern alle drei Spannungsmessungen unterschiedliche Messwerte. Der Betrag des Messwertes des negativen Abschnittes des biphasischen Strompulses ist aufgrund der höchsten Ladedauer am größten, der des ersten Abschnittes des Strompulses am zweitgrößten und der des dritten Abschnittes am kleinsten.

Im dritten in Abbildung 4 dargestellten Koordinatensystem wurden die auf den Messwert $U_3$ des kürzesten Abschnittes des biphasischen Strompulses bezogenen Messwerte über die bei jeder Spannungsmessung gegebenen zeitlichen Abstände $\Delta t$ vom Beginn der Ausgabe des jeweiligen Abschnittes des biphasischen Strompulses als Spannungsdifferenzen aufgetragen. Aufgrund dieser Differenzbildung ergibt sich die resultierende Spannungsdifferenz $\Delta U_3$ für den kürzesten Abschnitt des Strompulses in dem Koordinatensystem zu 0. Neben den drei Spannungsdifferenzen wurde in dem Koordinatensystem eine Ausgleichkurve eingetragen, die der bekannten Ladecharakteristik eines Kondensators folgt. Die den Verlauf dieser Ausgleichskurve bestimmenden Parameter $\tau$ und $U_0$ können als Ergebniswert der Lungenimpedanzmessung dienen. Alternativ oder ergänzend kann auch die Differenz der Ausgleichskurvenwerte für die zweite und dritte Messung bestimmt werden.

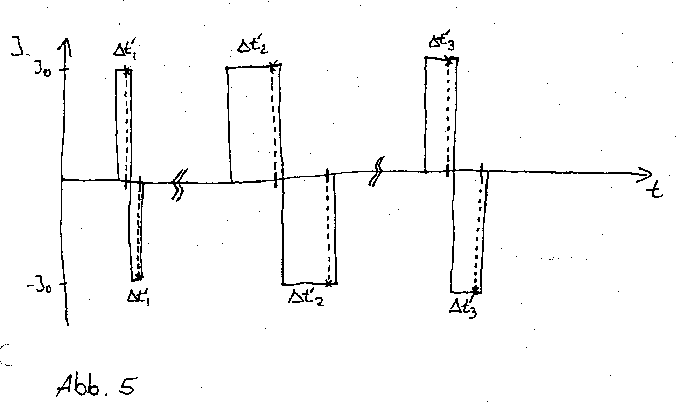

In Abbildung 5 ist ein weiterer zeitlicher Verlauf einer Folge von Strompulsen für die Bestimmung der Impedanz des zwischen den Elektroden liegenden Gewebes zu sehen. Auf der Ordinate sind die Zeitpunkte der Impedanzmessungen markiert.

Die Messung wird differentiell durchgeführt. D.h. es werden biphasische Strompulse verwendet, die aus zwei direkt aufeinander folgenden gleichlangen, aber gegenpoligen Strompulsen bestehen. Während jeder der beiden Strompulshälften wird jeweils eine Messung mit demselben zeitlichen Abstand zum Beginn der Abgabe der jeweiligen Strompulshälfte durchgeführt. Die Beträge der bei den beiden Teilmessungen bestimmten Spannungen werden addiert und das Zwischenergebnis halbiert. Auf diese Weise werden störende Gleichspannungen, die die Messung verfälschen könnten, eliminiert.

Die gezeigte Folge von biphasischen Strompulsen zeigt mehrere Strompulse unterschiedlicher Breite. Dabei wird pro biphasischem Strompuls nur eine differentielle Impedanzmessung bestehend aus zwei Teilmessungen durchgeführt. Um den kapazitiven Teil der Impedanz zu bestimmen, werden die Messergebnisse der Messungen für verschieden breite Strompulse bzw. verschieden lange seit Beginn der Abgabe des jeweiligen Strompulses verstrichene Zeiten wie bereits beschrieben ausgewertet. Alternativ können auch mehrere Messungen pro Strompulshälfte durchgeführt werden. Dann müssen die einzelnen Messungen gemäß ihrem zeitlichen Abstand zum Beginn der jeweiligen Strompulshälfte den entsprechenden Messungen der anderen Strompulshälfte zugeordnet werden, bevor die Mittelung zur Eliminierung störender Gleichspannungen und die Bestimmung des kapazitiven Anteils des Gewebeimpedanz durchgeführt wird.

**Patentansprüche**

1. Elektromedizinisches Implantat (100), insbesondere Herzschrittmacher, mit wenigstens
einem Messsignalgenerator (130), welches ausgebildet ist, Strompulse variabler Pulsdauer zu erzeugen und abzugeben
einer Impedanzmesseinheit (140) zur Bestimmung der Impedanz von menschlichem oder tierischem Gewebe insbesondere Lungengewebe,
einer Elektrodenanordnung (170, 180) mit mindestens zwei Elektroden (170, 180), die direkt oder mittelbar mit dem Messsignalgenerator (130) und der Impedanzmesseinheit (140) verbunden sind oder wenigstens zeitweilig verbunden werden können, oder mit einem Anschluss für eine solche Elektrodenanordnung (170, 180),
sowie einer Kontrolleinheit (120), die zum Steuern von Messsignalgenerator (130) und Impedanzmesseinheit (140) mit dem Messsignalgenerator (130) und der Impedanzmesseinheit (140) wenigstens mittelbar verbunden ist, und welche ausgebildet ist, zu wenigstens zwei unterschiedlichen Zeitpunkten den Messsignalgenerator (130) zu veranlassen, einen Strompuls zu erzeugen und abzugeben, so dass die Abgabe des ersten Strompulses vor dem Beginn der Abgabe des zweiten Strompulses beendet ist und die Pulsdauern der abgegebenen Strompulse sich voneinander unterscheiden,

und welche zudem ausgebildet ist, die Impedanzmesseinheit (140) zu veranlassen, die zwischen den mit dem Messsignalgenerator (130) und der Impedanzmesseinheit (140) verbundenen Elektroden (170, 180) anliegende Spannung zu Beginn einer bestimmten, mit dem Ende der Abgabe des jeweiligen Strompulses endenden Zeitspanne zu messen und als einen die gemessene Spannung repräsentierenden Spannungswert auszugeben,

**dadurch gekennzeichnet**,

die Kontrolleinheit (120) außerdem dazu ausgebildet ist,

für die Zeitpunkte jedes Veranlassens einer Spannungsmessung in Abhängigkeit eines ersten Parameters $U_0$ und eines zweiten Parameters $\tau$ eine Mehrzahl von Ausgleichskurvenwerten $U_A$ zu bestimmen, die sich gemäß der Formel

$$U_A = U_0 * (1 - e^{-t/\tau})$$

errechnen, wobei e die Eulersche Zahl und t die Dauer zwischen dem Zeitpunkt des Beginns der Abgabe des Strompulses, während dessen Abgabe die Kontrolleinheit (120) die Spannungsmessung veranlasst hat, für die die Mehrzahl von Ausgleichskurvenwerten $U_A$ bestimmt werden soll, und dem Zeitpunkt des Veranlassens der Spannungsmessung, für die die Mehrzahl von Ausgleichskurvenwerten $U_A$ bestimmt werden soll, sind,

für eine Mehrzahl von Kombinationen eines Wertes des ersten Parameters $U_0$ und eines Wertes des zweiten Parameters $\tau$ die Summe der Quadrate der Differenzen aller während der mehr als zwei Strompulse gemessenen Spannungswerte und des für jeden Spannungswert gemäß der jeweiligen Kombination eines Wertes des ersten Parameters $U_0$ und eines Wertes des zweiten Parameters $\tau$ errechneten Ausgleichskurvenwertes $U_A$ zu bestimmen, sowie durch Wahl der geringsten Summe eine Kombination eines Wertes des ersten Parameters $U_0$ und eines Wertes des zweiten Parameters $\tau$ zu bestimmen und als Ergebnisparameterkombination auszugeben.

2. Elektromedizinisches Implantat (100) nach Anspruch 1, **dadurch gekennzeichnet, dass** die Kontrolleinheit (120) dazu ausgebildet ist, einen eine Differenz zwischen den während zweier unterschiedlich langer vom Messsignalgenerator (130) abgegebener Strompulse durch die Impedanzmesseinheit (140) gemessenen Spannungswerten repräsentierenden Wert zu bestimmen und als Spannungsdifferenzwert auszugeben.

3. Elektromedizinisches Implantat (100) nach einem der Ansprüche 1 oder 2, **dadurch gekennzeichnet,**
**dass** die Kontrolleinheit (120) dazu ausgebildet ist, zu mehr als zwei unterschiedlichen Zeitpunkten den Messsignalgenerator (130) zu veranlassen, einen Strompuls zu erzeugen und abzugeben, so dass die Abgabe des ersten Strompulses vor dem Beginn der Abgabe des zweiten Strompulses beendet ist und die Pulsdauern der abgegebenen Strompulse sich voneinander unterscheiden,
und die Impedanzmesseinheit (140) zu veranlassen, die zwischen den mit dem Messsignalgenerator (130) und der Impedanzmesseinheit (140) verbundenen Elektroden (170, 180) anliegende Spannung zu Beginn einer bestimmten, mit dem Ende der Abgabe des jeweiligen Strompulses endenden Zeitspanne zu messen,
und **dass** die Kontrolleinheit (120) außerdem dazu ausgebildet ist, die Koeffizienten eines als Ausgleichskurve fungierenden Polynoms, für das die Summe der Quadrate der Differenzen aller während der mehr als zwei Strompulse gemessenen Spannungswerte und der Funktionswerte des Polynoms für die zeitlichen Abstände der Zeitpunkte des Veranlassens einer Spannungsmessung vom Zeitpunkt des Beginns der Ausgabe des jeweiligen Strompulses minimal ist, zu bestimmen und als Ergebniskoeffizienten auszugeben.

4. Elektromedizinisches Implantat (100) nach einem der Ansprüche 1 bis 3, **dadurch gekennzeichnet,**
**dass** der Messsignalgenerator (130) dazu ausgebildet ist, Strompulse variabler Pulsdauer zu erzeugen, wobei die Pulsdauer wenigstens eine erste Dauer und längstens eine zweite Dauer beträgt,
und **dass** die Kontrolleinheit (120) dazu ausgebildet ist, den Messsignalgenerator (130) zur Abgabe einer größeren Anzahl von Strompulsen vergleichsweise geringerer Pulsdauer als von Strompulsen mit vergleichsweise größerer Pulsdauer zu veranlassen.

5. Elektromedizinisches Implantat (100) nach einem der Ansprüche 1 bis 4, **dadurch gekennzeichnet, dass** die Kontrolleinheit (120) dazu ausgebildet ist, einen eine Differenz zwischen den Ausgleichskurvenwerten UA der Ergebnisparameterkombination bzw. der Ergebniskoeffizienten für die Dauern t der Strompulse mit der jeweils längsten und kürzesten Pulsdauer repräsentierenden Wert zu bestimmen und als Spannungsdifferenzwert auszugeben.

6. Elektromedizinisches Implantat (100) nach einem der vorhergehenden Ansprüche 1 bis 5, **dadurch gekennzeichnet, dass** der Messsignalgenerator (130) dazu ausgebildet ist, biphasische Strompulse zu erzeugen.

7. Elektromedizinisches Implantat (100) nach Anspruch 6, **dadurch gekennzeichnet, dass** die Impedanzmesseinheit (140) dazu ausgebildet ist, während der Abgaben unterschiedlich gepolter Abschnitte eines biphasischen Strompulses durch den Messsignalgenerator (130) Impedanzmessungen durchzuführen, und dass die Kontrolleinheit (120) dazu ausgebildet ist, die dabei bestimmten Messwerte zueinander in Bezug zu setzen und einen differentiellen Messwert zu bestimmen und auszugeben, der eine Differenz zwischen zwei während der Abgaben unterschiedlich gepolter Abschnitte eines biphasischen Strompulses bestimmten Messwerten repräsentiert.

8. Elektromedizinisches Implantat (100) nach einem der vorhergehenden Ansprüchen 1 bis 7, **dadurch gekennzeichnet,** **dass** der Messsignalgenerator (130) dazu ausgebildet ist, Sinusströme wenigstens zweier unterschiedlicher Frequenzen zu erzeugen und abzugeben, und **dass** die Impedanzmesseinheit (140) dazu ausgebildet ist, die Amplitude wenigstens einer Sinusspannung und/oder das Phasenverhältnis zwischen zwei Sinusspannungen zu messen und als einen die gemessene Amplitude bzw. das gemessene Phasenverhältnis oder beide repräsentierenden Spannungswert auszugeben.

9. Elektromedizinisches Implantat (100) nach Anspruch 8, **dadurch gekennzeichnet, dass** die Kontrolleinheit (120) dazu ausgebildet ist, den Messsignalgenerator (130) jeweils nach dem Verstreichen einer vorgebbaren Zeit solange zu veranlassen, die Frequenz des vom Messsignalgenerator (130) erzeugten und abgegebenen Sinusstromes zu erhöhen, bis die Impedanzmesseinheit (140) eine bestimmte, vorgebbare Phasendifferenz zwischen dem Sinusstrom und der an den mit der Impedanzmesseinheit (140) verbundenen Elektroden (170, 180) anliegenden Spannung feststellt und der Kontrolleinheit (120) signalisiert.

10. Elektromedizinisches Implantat (100) nach Anspruch 9, **dadurch gekennzeichnet, dass** die Kontrolleinheit (120) dazu ausgebildet ist, einen Zeitraum vorzugeben, über den die von der Impedanzmesseinheit (140) gemessene Phasendifferenz fortlaufend festgestellt werden soll, bevor die Impedanzmesseinheit (140) das Feststellen der Phasendifferenz signalisiert.

11. Elektromedizinisches Implantat (100) nach einem der Ansprüche 8 bis 10, **dadurch gekennzeichnet, dass** die Impedanzmesseinheit dazu ausgebildet ist, das Maximum und/oder das Minimum der gemessenen Phasendifferenzen zwischen dem Sinusstrom und der an den mit der Impedanzmesseinheit (140) verbundenen Elektroden (170, 180) anliegenden Spannung zu bestimmen und auszugeben.

12. Elektromedizinisches Implantat (100) nach einem der Ansprüche 8 bis 11, **dadurch gekennzeichnet,** **dass** die Impedanzmesseinheit (140) über ein Filter mit wenigstens einer Elektrode verbunden oder zu verbinden ist, wobei das Filter als Tiefpass- oder Bandpassfilter einen Durchlassbereich aufweist, der ein Eingangssignal der Frequenz des vom Messsignalgenerator (130) erzeugten Sinusstromes möglichst wenig dämpft.

13. Elektromedizinisches Implantat (100) nach Anspruch 12, **dadurch gekennzeichnet,** **dass** der Durchlassbereich des Filters kleiner ist als die Differenz der höchsten und der niedrigsten Frequenz der vom Messsignalgenerator (130) erzeugten Sinusströme und **dass** die Eckfrequenzen des Filters veränderbar sind und dergestalt vorgegeben werden können, dass die Frequenzen der jeweils vom Messsignalgenerator (130) erzeugten Sinusströme innerhalb des durch die vorgegebenen Eckfrequenzen bestimmten Durchlassbereichs des Filters liegen.

14. Elektromedizinisches Implantat (100) nach einem der Ansprüche 8 bis 13, **dadurch gekennzeichnet, dass** das elektromedizinische Implantat (100) über eine Subtrahierer verfügt, der mit der Impedanzmesseinheit (140) verbunden und dazu ausgebildet ist, einen eine Differenz wenigstens zweier durch die Impedanzmesseinheit (140) für Sinusströme unterschiedlicher Frequenzen bestimmter Amplituden repräsentierenden Wert zu bestimmen und als Amplitudendifferenzwert auszugeben.

15. Elektromedizinisches Implantat (100) nach einem der Ansprüche 8 bis 14, **dadurch gekennzeichnet, dass** der Messsignalgenerator (130) ausgebildet ist, einen Sinusstrom einer Frequenz von ungefähr 2 kHz und/oder einen Sinusstrom einer Frequenz von ungefähr 20 kHz zu erzeugen.

16. Elektromedizinisches Implantat (100) nach einem der vorangehenden Ansprüche 1 bis 15, **dadurch gekennzeichnet,**
**dass** der Impedanzmesseinheit (140) ein Verstärker (150) vorgeschaltet ist, der dazu ausgebildet ist, eine zwischen einer mit dem Messsignalgenerator (130) und einer über den Verstärker mittelbar mit der Impedanzmesseinheit (140) verbundenen Elektrode (170, 180) anliegende Spannung zu verstärken bzw. abzuschwächen und die resultierende Spannung der Impedanzmesseinheit (140) zuzuleiten,
wobei der Verstärkungsfaktor des Verstärkers (150) veränderbar ist.

17. Elektromedizinisches Implantat (100) nach Anspruch 16, **dadurch gekennzeichnet,**
**dass** der Verstärkungsfaktor des Verstärkers (150) im Betrieb dergestalt angepasst werden kann, dass der Betrag des Maximalwertes der verstärkten Spannung einen ersten Spannungswert nicht unterschreitet und einen zweiten Spannungswert nicht überschreitet.

18. Elektromedizinisches Implantat (100) nach einem der Ansprüche 16 oder 17, **dadurch gekennzeichnet, dass** dem Verstärker (150) eine Subtraktionseinheit (160) vorgeschaltet ist, die ausgebildet ist, einen vorgegebenen und einstellbaren Spannungswert von der an der Subtraktionseinheit (160) anliegenden Spannung abzuziehen und die resultierende Spannung dem Verstärker (150) zuzuführen.

19. Elektromedizinisches Implantat (100) nach einem der vorangehenden Ansprüche 1 bis 18, **dadurch gekennzeichnet,**
**dass** das elektromedizinische Implantat (100) über mehr als zwei Elektroden (170, 180) verfügt oder mit mehr als zwei Elektroden (170, 180) verbunden werden kann,
und **dass** die Impedanzmesseinheit (140) dazu ausgebildet ist, mit einer wählbaren Elektrode (170, 180) und einer feststehenden oder wählbaren Elektrode (170, 180) verbunden zu werden und eine zwischen diesen Elektroden (170, 180) anliegende Spannung zu messen.

20. Elektromedizinisches Implantat (100) nach einem der vorangehenden Ansprüche 1 bis 19, **dadurch gekennzeichnet,**
**dass** das elektromedizinische Implantat (100) über mehr als zwei Elektroden (170, 180) verfügt oder mit mehr als zwei Elektroden (170, 180) verbunden werden kann,
und **dass** der Messsignalgenerator (130) dazu ausgebildet ist, mit einer wählbaren Elektrode (170, 180) und einer feststehenden oder wählbaren Elektrode (170, 180) verbunden zu werden.

21. Elektromedizinisches Implantat (100) nach einem der Ansprüche 19 oder 20, **dadurch gekennzeichnet, dass** die Kontrolleinheit (120) dazu ausgebildet ist, für mehrere verschiedene Kombinationen zweier mit Impedanzmesseinheit (140) und Messsignalgenerator (130) verbundener oder zu verbindenden Elektroden (170, 180) den Messsignalgenerator (130) zum Abgeben eines Strompulses oder eines Sinusstromes und die Impedanzmesseinheit (140) zum Messen der zwischen der jeweiligen Kombination zweier Elektroden (170, 180) anliegenden Spannung zu veranlassen und einen Mittelwert der jeweils dabei bestimmten, die gemessenen Spannungen repräsentierenden Spannungswerte zu berechnen und auszugeben.

22. Elektromedizinisches Implantat (100) nach einem der vorangehenden Ansprüche 1 bis 21, **dadurch gekennzeichnet, dass** das elektromedizinische Implantat (100) ein Herzschrittmacher, Kardioverter oder Defibrillator ist.

23. Elektromedizinisches Implantat (100) nach einem der vorangehenden Ansprüche 1 bis 22, **dadurch gekennzeichnet,**
**dass** das elektromedizinische Implantat (100) über einen Herzaktivitätsdetektor verfügt, der ausgebildet ist, den Herzzyklus eines Patienten zu erfassen und zu einem bestimmten Zeitpunkt innerhalb eines Herzzyklus' ein Signal an die Kontrolleinheit (120) auszugeben,
und **dass** die Kontrolleinheit (120) dazu ausgebildet ist, den Messsignalgenerator (130) nach dem Empfangen eines Signals von dem Herzaktivitätsdetektor dazu zu veranlassen, einen Strompuls oder ein Messsignal zu erzeugen und über wenigstens eine Elektrode (170, 180) abzugeben.

24. Elektromedizinisches Implantat (100) nach einem der vorangehenden Ansprüche 1 bis 23, **dadurch gekennzeichnet,**

**dass** das elektromedizinische Implantat (100) über einen Atemaktivitätsdetektor verfügt, der ausgebildet ist, den Atemzyklus eines Patienten zu erfassen und zu einem bestimmten Zeitpunkt innerhalb eines Atemzyklus' ein Signal an die Kontrolleinheit (120) auszugeben,
und **dass** die Kontrolleinheit (120) dazu ausgebildet ist, den Messsignalgenerator (130) nach dem Empfangen eines Signals von dem Herzaktivitätsdetektor dazu zu veranlassen, einen Strompuls oder ein Messsignal zu erzeugen und über wenigstens eine Elektrode (170, 180) abzugeben.

25. Elektromedizinisches Implantat (100) nach einem der vorangehenden Ansprüche 1 bis 24, **dadurch gekennzeichnet,**
dass das elektromedizinische Implantat (100) dazu ausgebildet ist, Serien von Impedanzmessungen durchzuführen, und **dass** die Kontrolleinheit (120) dazu ausgebildet ist, den Mittelwert der Messergebnisse einer Serie von Impedanzmessungen zu bestimmen und als Ergebniswert auszugeben.

26. Elektromedizinisches Implantat (100) nach einem der vorangehenden Ansprüche 1 bis 25, **dadurch gekennzeichnet,**
dass das elektromedizinische Implantat (100) dazu ausgebildet ist, Serien von Impedanzmessungen durchzuführen, und **dass** die Kontrolleinheit (120) dazu ausgebildet ist, den Median der Messergebnisse einer Serie von Impedanzmessungen zu bestimmen und als Ergebniswert auszugeben.

27. Elektromedizinisches Implantat (100) nach einem der vorhergehenden Ansprüche 1 bis 26, **dadurch gekennzeichnet,**
dass das elektromedizinische Implantat (100) über eine drahtlose Datenübertragungsschnittstelle (110) verfügt und **dass** die drahtlose Datenübertragungsschnittstelle (110) dazu ausgebildet ist, einen oder mehrere der durch die Kontrolleinheit (120) bestimmten oder ausgegebenen Parameter wie Spannungsdifferenzwert, Mittelwert, Wert des ersten Parameters U0, Wert des zweiten Parameters $\tau$ und Amplitudendifferenzwert und/oder einen oder mehrere der von der Impedanzmesseinheit (140) bestimmten Spannungs- bzw. Amplitudenwerte über die drahtlose Datenübertragungsschnittstelle (110) direkt oder mittelbar als Patientendaten an ein Home Monitoring Service Center (300) zu senden.

28. System mit wenigstens
einem elektromedizinischen Implantat (100) nach Anspruch 27
und einem Patientengerät (200), einem Home Monitoring Service Center (300) oder beidem,
wobei das elektromedizinische Implantat (100) und das Patientengerät (200) über drahtlose Datenübertragungsschnittstellen (110, 210) und das Patientengerät (200) und das Home Monitoring Service Center (300) über drahtlose (230, 310) oder drahtgebundene Datenübertragungsschnittstelle miteinander verbunden sein können.

29. System nach Anspruch 28, **dadurch gekennzeichnet,**
dass das Patientengerät (200) oder das Home Monitoring Service Center (300) dazu ausgebildet ist, die von dem elektromedizinischen Implantat (100) empfangenen Patientendaten zu speichern und Kurzzeit- und Langzeitmittelwerte der einzelnen Patientendaten zu berechnen.

30. System nach Anspruch 29, **dadurch gekennzeichnet,**
dass das Patientengerät (200) oder das Home Monitoring Service Center (300) dazu ausgebildet ist, empfangene Patientendaten mit den berechneten Kurzzeit- und Langzeitmittelwerten zu vergleichen.

31. System nach Anspruch 30, **dadurch gekennzeichnet,**
dass das Patientengerät (200) oder das Home Monitoring Service Center (300) dazu ausgebildet ist, Abweichungen der mit den Kurzzeit- und Langzeitmittelwerten verglichenen Patientendaten von den Kurzzeit- und Langzeitmittelwerten anzuzeigen.

**Claims**

1. An electromedical implant (100) in particular a cardiac pacemaker, comprising at least
one measurement signal generator (130), which is designed to generate and output current pulses having a variable pulse duration,
one impedance measuring unit (140) for determining the impedance of human or animal tissue, in particular lung tissue,

one electrode arrangement (170, 180) comprising at least two electrodes (170, 180), which are directly or indirectly connected to the measurement signal generator (130) and the impedance measuring unit (140) or can be connected at least temporarily thereto, or comprising a connector for such an electrode arrangement (170, 180),
and comprising a control unit (120), which is connected at least indirectly to the measurement signal generator (130) and the impedance measuring unit (140) in order to control the measurement signal generator (130) and the impedance measuring unit (140), and which is designed to trigger the measurement signal generator (130) to generate and output a current pulse at at least two different times such that the output of the first current pulse is ended before the output of the second current pulse is started and the pulse durations of the current pulses that are output differ from one another,
and which is also designed to trigger the impedance measuring unit (140) to measure the voltage that is present between the electrodes (170, 180) that are connected to the measurement signal generator (130) and the impedance measuring unit (140) at the beginning of a certain time period that ends at the end of the output of the respective current pulse and to output this as a voltage value that represents the measured voltage,
**characterized in that**
the control unit (120) is also designed
to determine a plurality of compensating curve values $U_A$ for the times of each triggering of a voltage measurement as a function of a first parameter $U_0$ and a second parameter $\tau$, said compensating curve values being calculated according to the formula

$$U_A = U_0 * (1 - e^{-t/\tau})$$

wherein e is Euler's constant and t is the duration between the time of the beginning of the output of the current pulse, during the output of which the control unit (120) triggered the voltage measurement for which the majority of compensating curves $U_A$ is supposed to be determined, and the time of the triggering of the voltage measurement for which the majority of compensating curve values $U_A$ is supposed to be determined,
said control unit also being designed to determine, for a plurality of combinations of a value of the first parameter $U_0$ and a value of the second parameter $\tau$, the sum of the squares of the differences of all the voltage values measured during the more than two current pulses and the compensating curve value $U_A$ calculated for each voltage value according to the respective combination of a value of the first parameters $U_0$ and a value of the second parameters $\tau$,
and, by selecting the lowest sum, to determine a combination of a value of the first parameter $U_0$ and a value of the second parameter $\tau$ and to output this as a result parameter combination.

2.  The electromedical implant (100) according to claim 1, **characterized in that** the control unit (120) is designed to determine a value that represents a difference between the voltage values that are measured by the impedance measuring unit (140) during two current pulses that have different durations and are output by the measurement signal generator (130).

3.  The electromedical implant (100) according to any one of claims 1 or 2, **characterized in that**
the control unit (120) is designed to trigger the measurement signal generator (130) to generate and output a current pulse at more than two different times such that the output of the first current pulse is ended before the output of the second current pulse is started and the pulse durations of the current pulses that are output differ from one another,
and to trigger the impedance measuring unit (140) to measure the voltage that is present between the electrodes (170, 180) that are connected to the measurement signal generator (130) and the impedance measuring unit (140) at the beginning of a certain time period that ends at the end of the output of the respective current pulse,
and that the control unit (120) is also designed to determine the coefficients of a polynomial, which functions as a compensating curve and for which the sum of the squares of differences is minimal, specifically the difference between all the voltage values that are measured during the more than two current pulses and the function values of the polynomial for the time intervals of the times at which a voltage measurement was triggered and the time at which the output of the respective current pulse was started, and to output these as results coefficients.

4.  The electromedical implant (100) according to any one of claims 1 to 3, **characterized in that**
the measurement signal generator (130) is designed to generate current pulses having a variable pulse duration, wherein the pulse duration is a first duration at the least and a second duration at the most,
and that the control unit (120) is designed to trigger the measurement signal generator (130) to output a greater number of current pulses having a relatively shorter pulse duration than the number of current pulses having a relatively longer pulse duration.

5. The electromedical implant (100) according to any one of claims 1 to 4, **characterized in that** the control unit (120) is designed to determine a value that represents a difference between the compensating curve values $U_A$ of the result parameter combination or the result coefficients for the durations t of the current pulses having the respectively longest and shortest pulse durations, and to output this as the voltage difference value.

6. The electromedical implant (100) according to any one of claims 1 to 5, **characterized in that** the measurement signal generator (130) is designed to generate biphasic current pulses.

7. The electromedical implant (100) according to claim 6, **characterized in that** the impedance measuring unit (140) is designed to carry out impedance measurements during the outputs of differently polarized sections of a biphasic current pulse by the measurement signal generator (130),
and that the control unit (120) is designed to relate the determined measurement values to one another and to determine and output a differential measurement value that represents a difference between two measurement values that are determined during the outputs of differently polarized sections of a biphasic current pulse.

8. The electromedical implant (100) according to any one of the preceding claims 1 to 7, **characterized in that** the measurement signal generator (130) is designed to generate and output sinusoidal currents having at least two different frequencies,
and that the impedance measurement unit (140) is designed to measure the amplitudes of at least one sinusoidal voltage and/or the phase relationship between two sinusoidal voltages and output this as a voltage value that represents the measured amplitude or the measured phase relationship, or both.

9. The electromedical implant (100) according to claim 8, **characterized in that** the control unit (120) is designed such that, after each passage of a predefinable time, said control unit triggers the measurement signal generator (130) to increase the frequency of the sinusoidal current that is generated and output by the measurement signal generator (130) until the impedance measurement unit (140) detects a certain, predefinable phase difference between the sinusoidal current and the voltage that is present at the electrodes (170, 180) connected to the impedance measurement unit (140), and to signal the control unit (120).

10. The electromedical implant (100) according to claim 9, **characterized in that** the control unit (120) is designed to specify a period of time during which the phase difference measured by the impedance measuring unit (140) is supposed to be continuously determined before the impedance measuring unit (140) signals the detection of the phase difference.

11. The electromedical implant (100) according to any one of claims 8 to 10, **characterized in that** the impedance measuring unit is designed to determine and output the maximum and/or the minimum of the measured phase differences between the sinusoidal current and the voltage that is present at the electrodes (170, 180) connected to the impedance measuring unit (140).

12. The electromedical implant (100) according to any one of claims 8 to 11, **characterized in that** the impedance measuring unit (140) is connected or is supposed to be connected to at least one of the electrodes via a filter,
wherein the filter has a passband, as the low-pass or band-pass filter, which dampens the input signal of the frequency of the sinusoidal current generated by the measurement signal generator (130) to the least extent possible.

13. The electromedical implant (100) according to claim 12, **characterized in that** the passband of the filter is smaller than the difference between the highest and the lowest frequency of the sinusoidal currents generated by the measurement signal generator (130),
and that the cut-off frequencies of the filter can be changed and can be predefined such that the frequencies of each of the sinusoidal currents generated by the measurement signal generator (130) are located within the passband of the filter that is determined by the predefined cut-off frequencies.

14. The electromedical implant (100) according to any one of claims 8 to 13, **characterized in that** the electromedical implant (100) has a subtractor, which is connected to the impedance measuring unit (140) and is designed to determine a value that represents a difference between at least two amplitudes determined by the impedance measuring unit (140) for sinusoidal currents having different frequencies and to output this as the amplitude difference value.

15. The electromedical implant (100) according to any one of claims 8 to 14, **characterized in that** the measurement signal generator (130) is designed to generate a sinusoidal current having a frequency of approximately 2 kHz and/or a sinusoidal current having a frequency of approximately 20 kHz.

16. The electromedical implant (100) according to any one of the preceding claims 1 to 15, **characterized in that** installed upstream of the impedance measuring unit (140) is an amplifier (150), which is designed to amplify or attenuate a voltage that is present between an electrode (170, 180), respectively, that is connected to the measurement signal generator (130) and that is directly connected to the impedance measuring unit (140) via the amplifier, and to feed the resultant voltage to the impedance measuring unit (140),
wherein the amplification factor of the amplifier (150) can be changed.

17. The electromedical implant (100) according to claim 16, **characterized in that**
the amplification factor of the amplifier (150) can be adjusted during operation such that the amount of the maximum value of the amplified voltage does not fall below a first voltage value and does not exceed a second voltage value.

18. The electromedical implant (100) according to any one of claims 16 or 17, **characterized in that** installed upstream of the amplifier (150) is a subtraction unit (160), which is designed to subtract a predefined and adjustable voltage value from the voltage that is present at the subtraction unit (160) and feed the resultant voltage to the amplifier (150).

19. The electromedical implant (100) according to any one of the preceding claims 1 to 18, **characterized in that**
the electromedical implant (100) has more than two electrodes (170, 180) or can be connected to more than two electrodes (170, 180),
and that the impedance measurement unit (140) is designed to be connected to a selectable electrode (170, 180) and a fixed or selectable electrode (170, 180) and measure a voltage that is present between these electrodes (170, 180).

20. The electromedical implant (100) according to any one of the preceding claims 1 to 19, **characterized in that**
the electromedical implant (100) has more than two electrodes (170, 180) or can be connected to more than two electrodes (170, 180),
and that the measurement signal generator (130) is designed to be connected to a selectable electrode (170, 180) and to a fixed or selectable electrode (170, 180).

21. The electromedical implant (100) according to any one of claims 19 or 20, **characterized in that** the control unit (120) is designed such that, for a plurality of different combinations of two electrodes (170, 180) that are connected or are supposed to be connected to the impedance measuring unit (140) and the measurement signal generator (130), said control unit triggers the measurement signal generator (130) to output a current pulse or a sinusoidal current and triggers the impedance measuring unit (140) to measure the voltage that is present between the respective combination of two electrodes (170, 180), and such that said control unit calculates and outputs a mean value of the respective voltage values that are determined as a result and that represents the measured voltages.

22. The electromedical implant (100) according to any one of the preceding claims 1 to 21, **characterized in that** the electromedical implant (100) is a cardiac pacemaker, a cardioverter or a defibrillator.

23. The electromedical implant (100) according to any one of the preceding claims 1 to 22, **characterized in that**
the electromedical implant (100) comprises a cardiac activity detector, which is designed to detect the cardiac cycle of a patient and output a signal to the control unit (120) at a certain time within a cardiac cycle,
and that the control unit (120) is designed such that, after receipt of a signal from the cardiac activity detector, said control unit triggers the measurement signal generator (130) to generate a current pulse or a measurement signal and output this via at least one electrode (170, 180).

24. The electromedical implant (100) according to any one of the preceding claims 1 to 23, **characterized in that**
the electromedical implant (100) comprises a respiration activity detector, which is designed to detect the respiration cycle of a patient and output a signal to the control unit (120) at a certain time within a respiration cycle,
and that the control unit (120) is designed such that, after receipt of a signal from the cardiac activity detector, said control unit triggers the measurement signal generator (130) to generate a current pulse or a measurement signal and output this via at least one electrode (170, 180).

**25.** The electromedical implant (100) according to any one of the preceding claims 1 to 24, **characterized in that**
the electromedical implant (100) is designed to carry out series of impedance measurements,
and that the control unit (120) is designed to determine the mean value of the measurement results of a series of impedance measurements and output this as the result value.

**26.** The electromedical implant (100) according to any one of the preceding claims 1 to 25, **characterized in that**
the electromedical implant (100) is designed to carry out series of impedance measurements,
and that the control unit (120) is designed to determine the median of the measurement results of a series of impedance measurements and output this as the result value.

**27.** The electromedical implant (100) according to any one of the preceding claims 1 to 26, **characterized in that**
the electromedical implant (100) has a wireless data transmission interface (110),
and that the wireless data transmission interface (110) is designed to directly or indirectly send one or more of the parameters that are determined or output by the control unit (120), such as voltage difference value, mean value, value of the first parameters $U_0$, value of the second parameter $\tau$ and amplitude difference value and/or one or more of the voltage or amplitude values determined by the impedance measuring unit (140), as patient data, to a home monitoring service center (300).

**28.** A system comprising at least
one electromedical implant (100) according to claim 27
and one patient device (200), a home monitoring service center (300), or both,
wherein the electromedical implant (100) and the patient device (200) can be connected to one other via wireless data transmission interfaces (110, 210), and the patient device (200) and the home monitoring service center (300) can be connected to one another via a wireless (230, 310) or wired data transmission interface.

**29.** The system according to claim 28, **characterized in that**
the patient device (200) or the home monitoring service center (300) is designed to store the patient data received from the electromedical implant (100) and calculate short-term and long-term mean values of the individual patient data.

**30.** The system according to claim 29, **characterized in that**
the patient device (200) or the home monitoring service center (300) is designed to compare received patient data with the calculated short-term and long-term mean values.

**31.** The system according to claim 30, **characterized in that**
the patient device (200) or the home monitoring service center (300) is designed to display deviations of the patient data compared with the calculated short-term and long-term mean values from the short-term and long-term mean values.

**Revendications**

**1.** Implant électromédical (100), en particulier un stimulateur cardiaque, avec au moins
un générateur de signaux de mesure (130) conçu pour produire et délivrer des impulsions électriques de durée d'impulsion variable,
une unité de mesure d'impédance (140) destinée à déterminer l'impédance de tissu humain ou animal, en particulier d'un tissu pulmonaire,
un ensemble d'électrodes (170, 180) avec au moins deux électrodes (170, 180) reliées directement ou indirectement ou pouvant au moins être reliées temporairement au générateur de signaux de mesure (130) et à l'unité de mesure d'impédance (140), ou avec un raccordement pour un tel ensemble d'électrodes (170, 180),
ainsi qu'une unité de contrôle (120) reliée au moins indirectement au générateur de signaux de mesure (130) et à l'unité de mesure d'impédance (140) pour commander le générateur de signaux de mesure (130) et l'unité de mesure d'impédance (140), et conçue pour inciter, en au moins deux moments différents, le générateur de signaux de mesure (130) à produire et délivrer une impulsion électrique, de manière à ce que la délivrance de la première impulsion électrique soit terminée avant le début de la délivrance de la deuxième impulsion électrique, et à ce que les durées d'impulsion des impulsion électrique délivrées se différencient l'une de l'autre,
et conçue en outre pour inciter l'unité de mesure d'impédance (140) à mesurer la tension appliquée entre les électrodes (170, 180) reliées au générateur de signaux de mesure (130) et à l'unité de mesure d'impédance (140) au début d'un certain laps de temps finissant à la fin de la délivrance de l'impulsion électrique respective, et pour délivrer une valeur de tension représentant la tension mesurée,
**caractérisé en ce que**
l'unité de contrôle (120) est en outre conçue pour
déterminer une pluralité de valeur de courbe de compensation $U_A$ en fonction d'un premier paramètre U0 et d'un deuxième paramètre $\tau$, pour tous les moments d'incitation à une mesure de tension, laquelle est calculée selon la formule

$$U_A = U_0 * (1 - e^{-t/\tau})$$

où e représente le nombre eulérien et t la durée entre le moment du début de la délivrance de l'impulsion électrique, délivrance pendant laquelle l'unité de contrôle (120) a incité la mesure de tension pour laquelle la pluralité de valeurs de courbes de compensation $U_A$ doit être déterminée, et le moment d'incitation de mesure de tension, pour laquelle la pluralité de valeurs de courbes de compensation $U_A$ doit être déterminée,
déterminer, pour une pluralité de combinaisons d'une valeur du premier paramètre $U_0$ et d'une valeur du deuxième paramètre $\tau$, la somme des carrés des différences de toutes les valeurs de tension mesurées pendant les plus de deux impulsions électriques, et de la valeur de courbe de compensation $U_A$ calculée pour chaque valeur de tension selon la combinaison respective d'une valeur du premier paramètre $U_0$ et d'une valeur du deuxième paramètre $\tau$, et pour déterminer une combinaison d'une valeur du premier paramètre $U_0$ et une valeur du deuxième paramètre $\tau$, en choisissant la somme la plus petite, et la délivrer comme combinaison de paramètres de résultat.

**2.** Implant électromédical (100) selon la revendication 1, **caractérisé en ce que** l'unité de contrôle (120) est conçue pour déterminer une valeur représentant une différence entre les valeurs de tension mesurées par l'unité de mesure d'impédance (140) pendant deux impulsions électriques de différentes longueurs, délivrées par le générateur de signaux de mesure (130).

**3.** Implant électromédical (100) selon l'une des revendications 1 ou 2, **caractérisé en ce que**
l'unité de contrôle (120) est conçue pour inciter le générateur de signaux de mesure (130) à produire et délivrer une impulsion électrique en plus de deux instants, de telle façon que la délivrance de la première impulsion électrique se termine avant le début de la délivrance de la deuxième impulsion électrique, et les durées d'impulsion des impulsions électriques délivrées se différencient les unes des autres,
et pour inciter l'unité de mesure d'impédance (140) à mesurer la tension appliquée entre les électrodes (170, 180) reliées au générateur de signaux de mesure (130) et à l'unité de mesure d'impédance (140) au début d'un laps de temps déterminé, finissant à la fin de la délivrance de l'impulsion électrique respective,
et **en ce que** l'unité de contrôle (120) est en outre conçue pour déterminer les coefficients d'un polynôme servant de courbe de compensation, pour lequel la somme des carrés des différences de toutes les valeurs de tension mesurées pendant les plus de deux impulsions électriques et des valeurs de fonction du polynôme est minimale pour les écarts temporels entre les moments d'incitation d'une mesure de tension et le moment du début de la délivrance de l'impulsion électrique respective, et pour les délivrer comme coefficients de résultat.

**4.** Implant électromédical (100) selon l'une des revendications 1 à 3, **caractérisé en ce que**
le générateur de signaux de mesure (130) est conçu pour produire des impulsions électriques de durées d'impulsion variables, la durée d'impulsion s'élevant au moins à une première durée et au maximum à une deuxième durée,
et **en ce que** l'unité de contrôle (120) est conçue pour inciter le générateur de signaux de mesure (130) à délivrer un plus grand nombre d'impulsions électriques d'une durée d'impulsion comparativement inférieure que d'impulsions électriques de durée comparativement supérieure.

**5.** Implant électromédical (100) selon l'une des revendications 1 à 4, **caractérisé en ce que** l'unité de contrôle (120) est conçue pour déterminer une valeur représentant une différence entre les valeurs de courbes de compensation $U_A$ de la combinaison de paramètres de résultat ou des coefficients de résultat pour les durées t des impulsions électriques avec la durée d'impulsion respectivement la plus longue ou la plus courte, et pour la délivrer comme valeur de différence de tension.

**6.** Implant électromédical (100) selon l'une des revendications précédentes 1 à 5, **caractérisé en ce que** le générateur de signaux de mesure (130) est conçu pour produire des impulsions électriques biphasiques.

**7.** Implant électromédical (100) selon la revendication 6, **caractérisé en ce que** l'unité de mesure d'impédance (140) est conçue pour effectuer des mesures d'impédance pendant la délivrance de sections à pôles différents d'une impulsion électrique biphasique par le générateur de signaux de mesure (130),
et **en ce que** l'unité de contrôle (120) est conçue pour mettre en rapport entre elles les valeurs ainsi déterminées, et pour déterminer et délivrer une valeur de mesure différentielle, laquelle représente une différence entre deux valeurs de mesure déterminées pendant la délivrance de sections à pôles différents d'une impulsion électrique biphasique.

**8.** Implant électromédical (100) selon l'une des revendications précédentes 1 à 7, **caractérisé en ce que**
le générateur de signaux de mesure (130) est conçu pour produire et délivrer des courants sinusoïdaux présentant au moins deux fréquences différentes,
et **en ce que** l'unité de mesure d'impédance (140) est conçue pour mesurer l'amplitude d'au moins une tension sinusoïdale et/ou le rapport de phases entre deux tensions sinusoïdales, et pour les délivrer comme l'amplitude mesurée ou le rapport de phases mesuré ou une valeur de tension représentant les deux.

**9.** Implant électromédical (100) selon la revendication 8, **caractérisé en ce que** l'unité de contrôle (120) est conçue pour inciter le générateur de signaux de mesure (130), respectivement après l'écoulement d'un temps prédéfinissable, à augmenter la fréquence du courant sinusoïdal produit et délivré par le générateur de signaux de mesure (130), jusqu'à ce que l'unité de mesure d'impédance (140) détecte une différence de phases déterminée et prédéfinissable entre le courant sinusoïdal et la tension appliquée aux électrodes (170, 180) reliées à l'unité de mesure d'impédance (140), et la transmette à l'unité de contrôle (120).

**10.** Implant électromédical (100) selon la revendication 9, **caractérisé en ce que** l'unité de contrôle (120) est conçue pour prédéfinir une durée sur laquelle la différence de phases mesurée par l'unité de mesure d'impédance (140) doit être détectée en continu, avant que l'unité de mesure d'impédance (140) transmette la détection de la différence de phase.

**11.** Implant électromédical (100) selon l'une des revendications 8 à 10, **caractérisé en ce que** l'unité de mesure d'impédance est conçue pour déterminer et délivrer le maximum et/ou le minimum des différences de phases entre le courant sinusoïdal et la tension appliquée aux électrodes (170, 180) reliées à l'unité de mesure d'impédance (140).

**12.** Implant électromédical (100) selon l'une des revendications 8 à 11, **caractérisé en ce que**
l'unité de mesure d'impédance (140) est reliée ou destinée à être reliée à au moins une électrode par le biais d'un filtre, dans lequel le filtre en tant que filtre passe-bas ou passe-bande présente une région de passage atténuant le moins possible un signal d'entrée de la fréquence du courant sinusoïdal produit par le générateur de signaux de mesure (130).

**13.** Implant électromédical (100) selon la revendication 12, **caractérisé en ce que**
la région de passage du filtre est plus petite que la différence entre la fréquence la plus élevée et la fréquence la moins élevée des courants sinusoïdaux produits par le générateur de signaux de mesure (130),
et **en ce que** les fréquences de coupure du filtre sont modifiables et peuvent être prédéfinies de manière à ce que les fréquences des courants sinusoïdaux respectivement produits par le générateur de signaux de mesure (130) se trouvent dans la région de passage du filtre déterminée par les fréquences de coupure prédéfinies.

**14.** Implant électromédical (100) selon l'une des revendications 8 à 13, **caractérisé en ce que** l'implant électromédical (100) dispose d'un soustracteur relié à l'unité de mesure d'impédance (140) et conçu pour déterminer une valeur représentant une différence entre au moins deux amplitudes déterminées par l'unité de mesure d'impédance (140) pour des courants sinusoïdaux présentant des fréquences différentes, et pour la délivrer comme valeur de différence d'amplitude.

**15.** Implant électromédical (100) selon l'une des revendications 8 à 14, **caractérisé en ce que** le générateur de signaux de mesure (130) est conçu pour produire un courant sinusoïdal présentant une fréquence d'environ 2 kHz et/ou un courant sinusoïdal présentant une fréquence d'environ 20 kHz.

**16.** Implant électromédical (100) selon l'une des revendications précédentes 1 à 15, **caractérisé en ce que**
un amplificateur (150) est raccordé en amont de l'unité de mesure d'impédance (140), lequel est conçu pour amplifier ou réduire une tension appliquée entre une électrode (170, 180) reliée au générateur de signaux de mesure (130) et une électrode reliée indirectement à l'unité de mesure d'impédance (140) par le biais de l'amplificateur, et pour fournir la tension résultante à l'unité de mesure d'impédance (140),
dans lequel le facteur d'amplification de l'amplificateur (150) est modifiable.

17. Implant électromédical (100) selon la revendication 16, **caractérisé en ce que**
pendant le fonctionnement, le facteur d'amplification de l'amplificateur (150) peut être adapté de manière à ce que le montant de la valeur maximale de la tension amplifiée ne tombe pas en dessous d'une première valeur de tension et ne dépasse pas une deuxième valeur de tension.

18. Implant électromédical (100) selon l'une des revendications 16 ou 17, **caractérisé en ce qu'**une unité de soustraction (160) est raccordée en amont de l'amplificateur (150), laquelle est conçue pour soustraire une valeur de tension prédéfinie et réglable de la tension appliquée à l'unité de soustraction, et pour fournir la tension résultante à l'amplificateur (150).

19. Implant électromédical (100) selon l'une des revendications précédentes 1 à 18, **caractérisé en ce que**
l'implant électromédical (100) dispose de plus de deux électrodes (170, 180) ou peut être relié à plus de deux électrodes (170, 180),
et **en ce que** l'unité de mesure d'impédance (140) est conçue pour être reliée à une électrode (170, 180) au choix et à une électrode (170, 180) au choix ou fixe, et pour mesurer une tension appliquée entre ces électrodes (170, 180).

20. Implant électromédical (100) selon l'une des revendications précédentes 1 à 19, **caractérisé en ce que**
l'implant électromédical (100) dispose de plus de deux électrodes (170, 180) ou peut être relié à plus de deux électrodes (170, 180),
et **en ce que** le générateur de signaux de mesure (130) est conçu pour être relié à une électrode (170, 180) au choix et à une électrode (170, 180) au choix ou fixe.

21. Implant électromédical (100) selon l'une des revendications 19 ou 20, **caractérisé en ce que** l'unité de contrôle (120) est conçue pour inciter le générateur de signaux de mesure (130) à délivrer une impulsion électrique ou un courant sinusoïdal et pour inciter l'unité de mesure d'impédance (140) à mesurer la tension appliquée entre la combinaison respective de deux électrodes (170, 180), pour plusieurs combinaisons différentes de deux électrodes (170, 180) reliées ou aptes à être reliées à l'unité de mesure d'impédance (140) et au générateur de signaux de mesure (130), et pour calculer et délivrer une valeur moyenne des valeurs de tension respectivement déterminées, représentant les tensions mesurées.

22. Implant électromédical (100) selon l'une des revendications précédentes 1 à 21, **caractérisé en ce que** l'implant électromédical (100) est un stimulateur cardiaque, un cardioverteur ou un défibrillateur.

23. Implant électromédical (100) selon l'une des revendications précédentes 1 à 22, **caractérisé en ce que**
l'implant électromédical (100) dispose d'un détecteur d'activité cardiaque conçu pour détecter le cycle cardiaque d'un patient et pour délivrer un signal à l'unité de contrôle (120) à un moment déterminé dans un cycle cardiaque,
et **en ce que** l'unité de contrôle (120) est conçue inciter le générateur de signaux de mesure (130) à produire une impulsion électrique ou un signal de mesure après la réception d'un signal venant du détecteur d'activité cardiaque, et pour la/le délivrer par le biais d'au moins une électrode (170, 180).

24. Implant électromédical (100) selon l'une des revendications précédentes 1 à 23, **caractérisé en ce que**
l'implant électromédical (100) dispose d'un détecteur d'activité respiratoire conçu pour détecter le cycle respiratoire d'un patient et pour délivrer un signal à l'unité de contrôle (120) à un moment donné dans le cycle respiratoire,
et **en ce que** l'unité de contrôle (120) est conçue pour inciter le générateur de signaux de mesure (130) à produire une impulsion électrique ou un signal de mesure, après la réception d'un signal venant du détecteur d'activité cardiaque, et pour le délivrer par le biais d'au moins une électrode (170, 180).

25. Implant électromédical (100) selon l'une des revendications précédentes 1 à 24, **caractérisé en ce que**
l'implant électromédical (100) est conçu pour effectuer des séries de mesures d'impédance,
et **en ce que** l'unité de contrôle (120) est conçue pour déterminer la valeur moyenne des résultats de mesure d'une série de mesures d'impédance, et pour la délivrer comme valeur de résultat.

26. Implant électromédical (100) selon l'une des revendications précédentes 1 à 25, **caractérisé en ce que**
l'implant électromédical (100) est conçu pour effectuer des séries de mesures d'impédance,
et **en ce que** l'unité de contrôle (120) est conçue pour déterminer le médian des résultats de mesure d'une série

**EP 1 665 983 B1**

de mesures d'impédance, et pour le délivrer comme valeur de résultat.

27. Implant électromédical (100) selon l'une des revendications précédentes 1 à 26, **caractérisé en ce que** l'implant électromédical (100) dispose d'une interface de transmission de données (110) sans fil, et **en ce que** l'interface de transmission de données (110) sans fil est conçue pour envoyer un ou plusieurs paramètres déterminés ou délivrés par l'unité de contrôle (120), tels que la valeur de la différence de tension, la valeur moyenne, la valeur du premier paramètre $U_0$, la valeur du deuxième paramètre $\tau$ et la valeur de la différence d'amplitude, et/ou une ou plusieurs des valeurs de tension ou d'amplitude déterminées par l'unité de mesure d'impédance (140), directement ou indirectement par le biais de l'interface de transmission de données (110) sans fil, en tant que données de patient, à un Home Monitoring Service Center (300).

28. Système avec au moins
un implant électromédical (100) selon la revendication 27,
un appareil pour patient (200), un Home Monitoring Service Center (300) ou les deux,
dans lequel l'implant électromédical (100) et l'appareil pour patient (200) peuvent être reliés entre eux par des interfaces de transmission de données sans fil (110, 210), et l'appareil pour patient (200) et le Home Monitoring Service Center (300) peuvent être reliés entre eux par une interface de transmission de données sans fil (230, 310) ou avec fil.

29. Système selon la revendication 28, **caractérisé en ce que**
l'appareil pour patient (200) ou le Home Monitoring Service Center (300) est conçu pour enregistrer les données de patient reçues par l'implant électromédical (100) et pour calculer des valeurs moyennes de court et de long terme des différentes données de patient.

30. Système selon la revendication 29, **caractérisé en ce que**
l'appareil pour patient (200) ou le Home Monitoring Service Center (300) est conçu pour comparer des données de patient reçues avec les valeurs moyennes de court et long terme calculées.

31. Système selon la revendication 30, **caractérisé en ce que**
l'appareil pour patient (200) ou le Home Monitoring Service Center (300) est conçu pour indiquer des écarts entre les données de patient comparées avec les valeurs moyennes de court et long terme et les valeurs moyennes de court et long terme.

Abb. 1

Abb.2

Abb.3

Abb. 4

Abb. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6473640 B
- US 5957861 A
- US 20040102712 A
- US 6553262 B
- US 5752976 A

**In der Beschreibung aufgeführte Nicht-Patentliteratur**

- **PALKO.** Multifrequency Device for Measurement of the Complex Electrical Bio-Impedance - Design and Application. *Proceedings RC IEEE-EMBS & 14th BMESI,* 1995, (45